# EUROPEAN PATENT SPECIFICATION

(11) **EP 2 049 482 B1**
(45) Date of publication and mention of the grant of the patent: **21.10.2015**
(21) Application number: 06795612.8
(22) Date of filing: 08.08.2006
(51) Int. Cl.: C07D 209/12, C07D 491/04, C07D 231/12

(54) **POSITIVELY CHARGED WATER-SOLUBLE PRODRUGS OF ARYL- AND HETEROARYLACETIC ACIDS WITH VERY FAST SKIN PENETRATION RATE**
POSITIV GELADENE, WASSERLÖSLICHE PRODRUGS VON ARYL- UND HETEROARYLESSIGSÄUREN MIT SEHR SCHNELLER HAUTPENETRATIONSGESCHWINDIGKEIT
PROMÉDICAMENTS HYDROSOLUBLES CHARGÉS POSITIVEMENT D'ACIDES ARYL- ET HETEROARYLACETIQUES AVEC UNE VITESSE DE PÉNÉTRATION DE LA PEAU TRÈS RAPIDE

(43) Date of publication of application: 22.04.2009
(73) Proprietor: Techfields Biochem Co. Ltd, Shanghai N/A 200444 (CN); Yu, Chongxi, Plainfield, Illinois 60585 (US)
(72) Inventor: YU, Chongxi, Illinois 60585 (US); XU, Lina, Shanghai N/a 200444 (CN)
(74) Representative: Viering, Jentschura & Partner Patent- und Rechtsanwälte
(86) International application number: PCT/IB2006/052732
(87) International publication number: WO 2008/017903

(56) References cited:
- WO-A1-02/068377
- WO-A1-03/029187
- WO-A2-01/58852
- FR-A- 1 593 024
- FR-A1- 2 410 641
- VENUTI M C ET AL: "SYNTHESIS AND BIOLOGICAL EVALUATION OF OMEGA-(N,N,N-TRIALKYLAMMONIUM) ALKYL ESTERS AND THIOESTERS OF CARBOXYLIC ACID NONSTEROIDAL ANTIINFLAMMATORY AGENTS" PHARMACEUTICAL RESEARCH, KLUWER ACADEMIC PUBLISHERS, NEW YORK, NY, US LNKD- DOI:10.1023/A:1015960522189, vol. 6, no. 10, 1 January 1989 (1989-01-01), pages 867-873, XP001098334 ISSN: 0724-8741
- SONG N. ET AL: 'Synthesis of a derivative quaternary ammonium-ibuprofen' JOURNAL OF OCEAN UNIVERSITY OF QINGDAO vol. 32, no. 6, 2002, pages 911 - 913, XP001539131

## Description

### Technical Field

The present invention relates to the preparations of positively charged and watersoluble pro-drugs of aryl- and heteroarylacetic acids and their medicinal use in a method for treating any nonsteroidal anti-inflammatory drugs (NSAIAs)-treatable conditions in humans or animals. More specifically, the present invention is to overcome the side effects that are associated with the use of NSAIAs. These pro-drugs can be administered orally or transdermally.

### Background Art

1-Methyl-5-(4-methylbenzoyl)-1H-pyrrole-2-acetic acid (tolmetin), 5-(4-Chlorobenzoyl)-1,4-dimethyl-1H-pyrrole-2-acetic acid (zomepirac), 1,8-diethyl-1,3,4,9-tetrahydropyrano-[3,4-b]indole-1-acetic acid (etodolac), 2-amino-3-benzoylbenzeneacetic acid (amfenac), 2-amino-3-(4-bromo-benzoyl) benzeneacetic acid (bromofenac), 3-chloro-4-(2-propenyloxy) benzeneacetic acid (alclofenac), 2-(2,4-dichlorophenoxy)benzeneacetic acid (fenclofenac), 1-(4-chlorobenzoyl-5-methoxy-2-methyl-1H-indole-3-acetic acid carboxymethyl ester (acemetacin), 4-(4-chlorophenyl)-2-phenyl-5-thiazoleacetic acid (fentiazac), 1-(4-chlorobenzoyl)-5-methoxy-2-methyl-1H-indole-3-acetic acid (indomethacin), 5-fluoro-2-methyl-1-[[(4-methylsulfmyl) phenyl]methylene]-1H-indene-3-acetic acid (sulindac), 3-(4-chlorophenyl)-1-phenyl-1H-pyrazole-4-acetic acid (lonazolac), [(1-benzyl-1H-indazol-3-yl)oxy]acetic acid (bendazac), 6-methoxyl-2-naphthalene-2-acetic acid (6MNA), p-isobutylphenylacetic acid (ibufenac), and related compounds are members of aryl- and heteroarylacetic acid group of nonsteroidal anti-inflammatory drugs. They are used for the relief of signs and symptoms of rheumatoid arthritis and osteoarthritis and for the treatment of dysmenorrheal. They are also used for the treatment of acute gouty arthritis and ankylosing spondylitis. They may be used for the treatment of dementia (McGeer; Patrick L. et al. U.S. Pat. No.5,192,753).

Unfortunately, a number of side effects are associated with the use of tolmetin, zomepirac, etodolac, amfenac, bromofenac, alclofenac, fenclofenac, acemetacin, fentiazac, indomethacin, sulindac, lonazolac, bendazac, 6MNA, ibufenac, and related compounds, most notably GI disturbances such as dyspepsia, gastroduodenal bleeding, gastric ulcerations, and gastritis. Fishman (Fishman; Robert, U.S. Pat. No. 7,052,715) indicated that an additional problem associated with oral medications is that the concentration levels which must be achieved in the blood stream must be significant in order to effectively treat distal areas of pain or inflammation. These levels are often much higher than would be necessary if it were possible to accurately target the particular site of pain or injury. Fishman and many others (Van Engelen et al. U.S. Pat. No. 6,416,772; Macrides et al. U.S. Pat. No. 6,346,278; Kirby et al. U.S. Pat. No. 6,444,234, Roentsch, et al., U.S. Pat. No. 5,654,337, Park, et al., U.S. Pat. No. 6,190,690, Pearson et al. U.S. Pat. No. 6,528,040, and Botknecht et al. U.S. Pat. No. 5,885,597) have tried to develop a delivery system for transdermal application by formulation. It is very difficult, however, to deliver therapeutically effective plasma levels of these kinds of drugs into the host by formulation, due to the slow skin penetration rate. Susan Milosovich, et. al. designed and prepared testosteronyl-4-dimethylaminobutyrate.HCl (TSBH), which has a lipophilic portion and a tertiary amine group that exists in the protonated form at physiological pH. They found that the prodrug (TSBH) diffuses through human skin ∼60 times faster than the drug (TS) itself [Susan Milosovich, et al., J. Pharm. Sci., 82, 227(1993).

### Disclosure of Invention

### Technical Problem

Tolmetin, zomepirac, etodolac, amfenac, bromofenac, alclofenac, fenclofenac, acemetacin, fentiazac, indomethacin, sulindac, lonazolac, bendazac, 6MNA, ibufenac, and related compounds, have been used medicinally for many years. They are used for the relief of signs and symptoms of rheumatoid arthritis and osteoarthritis and for the treatment of dysmenorrhea.

Unfortunately, a number of side effects are associated with the use of tolmetin, zomepirac, etodolac, amfenac, bromofenac, alclofenac, fenclofenac, acemetacin, indomethacin, sulindac, fentiazac, lonazolac, bendazac, 6MNA, ibufenac, and related compounds, most notably GI disturbances such as dyspepsia, gastroduodenal bleeding, gastric ulcerations, and gastritis. They are not soluble in aqueous solution and gastric juice. They stay in the GI tract for a long time and thus, may cause gastric mucosal cell damage.

### Technical Solution

This invention relates to the preparation of novel positively charged pro-drugs of tolmetin, zomepirac, etodolac, amfenac, bromofenac, indomethacin, sulindac, alclofenac, fenclofenac, acemetacin, fentiazac, lonazolac, bendazac, 6MNA, ibufenac and related compounds and their medicinal use. These pro-drugs are:
Diethylaminoethyl 1-(p-chlorobenzoyl)-5-methoxy-2-methylindole 3-acetate.AcOH,
Diethylaminoethyl (Z)-5-fluoro-2-methyl-1-[(4-methylsulfinyl) phenylmethylene]-1H-indene-3-acetate.AcOH,
Diethylaminoethyl 1-methyl-5-(4-methylbenzoyl)-1H-pyrrole-2-acetate.AcOH,
Diethylaminoethyl 5-(4-Chlorobenzoyl)-1,4-dimethyl-1H-pyrrole-2-acetate.AcOH,
Diethylaminoethyl 1,8-diethyl- 1,3,4,9-tetrahydropyrano-[3,4-b]indole-1-acetate.AcOH,
Diethylaminoethyl 2-amino-3-benzoylbenzeneacetate.AcOH,
Diethylaminoethyl 2-amino-3-(4-bromo-benzoyl)benzeneacetate.AcOH,
Diethylaminoethyl 3-chloro-4-(2-propenyloxy)benzeneacetate.AcOH,
Diethylaminoethyl 2-(2,4-dichlorophenoxy)benzeneacetate.AcOH,
Diethylaminoethyl 1-(4-chlorobenzoyl-5-methoxy-2-methyl-1H-indole-3-acetoxyacetate.AcOH,
Diethylaminoethyl 4-(4-chlorophenyl)-2-phenyl-5-thiazoleacetate.AcOH,
Diethylaminoethyl 3-(4-chlorophenyl)-1-phenyl-1H-pyrazole-4-acetate.AcOH,
Diethylaminoethyl [(1-benzyl- 1H-indazol-3-yl)oxy]acetate.AcOH,
Diethylaminoethyl 6-methoxyl-2-naphthalene-2-acetate.AcOH, and
Diethylaminoethyl p-isobutylphenylacetate.AcOH.

Drug absorption, whether from the gastrointestinal tract or other sites, requires the passage of the drug in a molecular form across the barrier membrane. The drug must first dissolve, and if the drug possesses the desirable biopharmaceutical properties, it will pass from a region of high concentration to a region of low concentration across the membrane into the blood or general circulation. All biological membranes contain lipids as major constituents. The molecules that play the dominant roles in membrane formation all have phosphate-containing highly polar head groups, and, in most cases, two highly hydrophobic hydrocarbon tails. Membranes are bilayers, with the hydrophilic head groups facing outward into the aqueous regions on either side. Very hydrophilic drugs cannot pass through the hydrophobic layer of membrane and very hydrophobic drugs will stay in the hydrophobic layer as part of the membrane due to their similarities and cannot enter the cytosol efficiently.

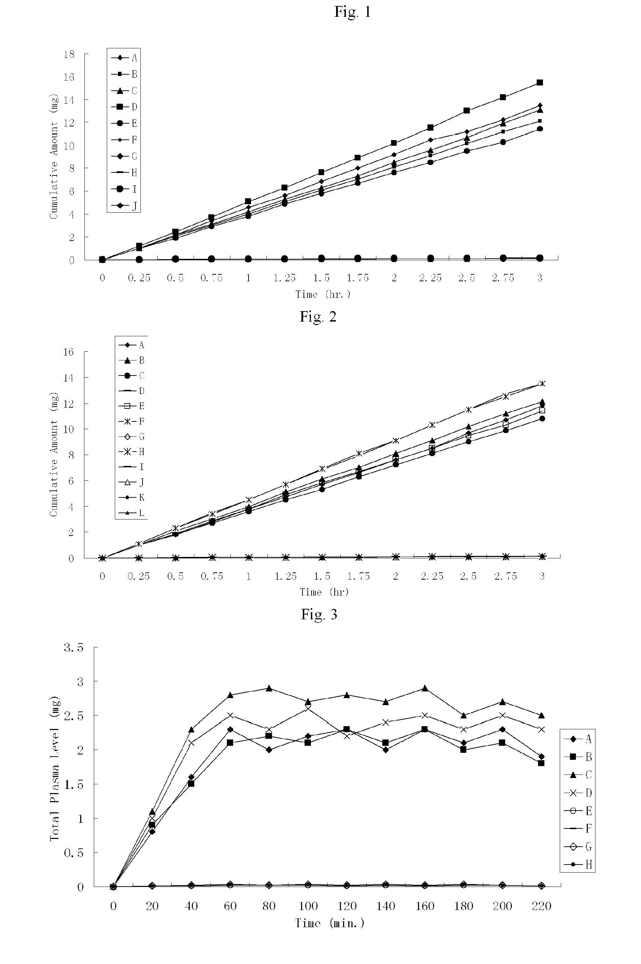

The goal of this invention is to avoid the side effects of tolmetin, zomepirac, etodolac, indomethacin, sulindac, amfenac, bromofenac, alclofenac, fenclofenac, acemetacin, fentiazac, lonazolac, bendazac, 6MNA, ibufenac, and related compounds by increasing their solubility in gastric juice and their penetration rate through the membrane and skin barrier which will make it administrable transdermally (topical application). These novel pro-drugs have two structural features in common: they have a lipophilic portion and a primary, secondary, or tertiary amine group that exists in the protonated form (hydrophilic part) at physiological pH. Such a hydrophilic-lipophilic balance is required for efficient passage through the membrane barrier [Susan Milosovich, et al., J. Pharm. Sci., 82, 227(1993)]. The positively charged amino groups largely increase the solubility of the drugs. The solubility of diethylaminoethyl 1-(p-chlorobenzoyl)-5-methoxy-2-methylindole 3-acetate.AcOH, diethylaminoethyl (Z)-5-fluoro-2-methyl-1-[(4-methylsulfinyl) phenylmethylene] - 1H-indene-3-acetate.AcOH, diethylaminoethyl 1-methyl-5-(4-methylbenzoyl)-1H-pyrrole-2-acetate.AcOH, diethylaminoethyl 5-(4-Chlorobenzoyl)-1,4-dimethyl-1H-pyrrole-2-acetate.AcOH, diethylaminoethyl 1,8-diethyl-1,3,4,9-tetrahydropyrano-[3,4-b]indole-1-acetate.AcOH, diethylaminoethyl 2-amino-3-benzoylbenzeneacetate.AcOH, diethylaminoethyl 2-amino-3-(4-bromo-benzoyl)benzeneacetate.AcOH, diethylaminoethyl 3-chloro-4-(2-propenyloxy)benzeneacetate.AcOH, diethylaminoethyl 2-(2,4-dichlorophenoxy)benzeneacetate.AcOH, diethylaminoethyl 1-(4-chlorobenzoyl-5-methoxy-2-methyl-1H-indole-3-acetoxyacetate.AcOH, diethylaminoethyl4-(4-chlorophenyl)-2-phenyl-5-thiazoleacetate.AcOH, diethylaminoethyl 3-(4-chlorophenyl)-1-phenyl-1H-pyrazole-4-acetate.AcOH, diethylaminoethyl [(1-benzyl-1H-indazol-3-yl)oxy]acetate.AcOH, diethylaminoethyl 6-methoxyl-2-naphthalene-2-acetate.AcOH, diethylaminoethyl p-isobutylphenylacetate.AcOH, 1-(p-chlorobenzoyl)-5-methoxy-2-methylindole 3-acetic acid (indomethacin), (Z)-5-fluoro-2-methyl-1-[(4-methylsulfinyl) phenylmethylene] - 1H-indene-3-acetic acid (sulindac), 1-methyl-5-(4-methylbenzoyl)-1H-pyrrole-2-acetic acid (tolmetin), 5-(4-Chlorobenzoyl)-1,4-dimethyl-1H-pyrrole-2-acetic acid (zomepirac), 1,8-diethyl-1,3,4,9-tetrahydropyrano-[3,4-b]indole-1-acetic acid (etodolac), 2-amino-3-benzoylbenzeneacetic acid (amfenac), 2-amino-3-(4-bromo-benzoyl)benzeneacetic acid (bromofenac), 3-chloro-4-(2-propenyloxy)benzeneacetic acid (alclofenac), 2-(2,4-dichlorophenoxy)benzeneacetic acid (fenclofenac), 1-(4-chlorobenzoyl-5-methoxy-2-methyl-1H-indole-3-acetic acid carboxymethyl ester (acemetacin), 4-(4-chlorophenyl)-2-phenyl-5-thiazoleacetic acid (fentiazac), 3-(4-chlorophenyl)-1-phenyl-1H-pyrazole-4-acetic acid (lonazolac), [(1-benzyl-1H-indazol-3-yl)oxy]acetic acid (bendazac), 6-methoxyl-2-naphthalene-2-acetic acid (6MNA), and p-isobutylphenylacetic acid (ibufenac) in water are >450, >400, >450, >450, >350, >450, >400, >450, >400, >450, >350, >400, >350, >400, >350, 0.1, 0.1, 0.2, 0.2, 0.1, 0.2, 0.1, 0.1, 0.1, 0.1, 0.1, 0.1, 0.1, 0.1 and 0.1 mg/ml, respectively. In many instances, the lowest or rate-limiting step in the sequence is the dissolution of the drug. Tolmetin, zomepirac, etodolac, amfenac, bromofenac, alclofenac, fenclofenac, indomethacin, sulindac, acemetacin, fentiazac, lonazolac, bendazac, 6MNA, ibufenac, and related compounds have a very low solubility in gastric juice. They stay in the GI tract for a long time and thus, may cause gastric mucosal cell damage. When these new pro-drugs are administered orally in a dosage form such as a tablet, capsule, solution, or suspension, they will dissolve in the gastric juice immediately. The positive charge on the amino groups of these pro-drugs will bond to the negative charge on the phosphate head group of the membrane. Thus, the local concentration outside of the membrane will be very high and will facilitate the passage of these pro-drugs from a region of high concentration to a region of low concentration. When these pro-drugs enter the membrane, the hydrophilic part will push the pro-drug into the cytosol, a semi-liquid concentrated aqueous solution or suspension. Due to the short stay in GI tract, the pro-drugs will not cause gastric mucosal cell damage. The penetration rates of diethylaminoethyl 1-(p-chlorobenzoyl)-5-methoxy-2-methylindole 3-acetate.AcOH, diethylaminoethyl5-fluoro-2-methyl-1-[(4-methylsulfinyl) phenylmethylene]-1H-indene-3-acetate.AcOH, diethylaminoethyl 1-methyl-5-(4-methylbenzoyl)-1H-pyrrole-2-acetate.AcOH, diethylaminoethyl 5-(4-Chlorobenzoyl)-1,4-dimethyl-1H-pyrrole-2-acetate.AcOH, diethylaminoethyl 1,8-diethyl-1,3,4,9-tetrahydropyrano-[3,4-b]indole-1-acetate.AcOH, [(1-benzyl-1H-indazol-3-yl)oxy]acetate.AcOH, 3-(4-chlorophenyl)-1-phenyl-1H-pyrazole-4-acetate.AcOH, [(1-benzyl-1H-indazol-3-yl)oxy]acetate.AcOH, indomethacin, sulindac, tolmetin, zomepirac, etodolac, amfenac, bromofenac, alclofenac, fenclofenac, acemetacin, fentiazac, lonazolac, bendazac, and related compounds through human skin were measured in vitro by using modified Franz cells, which were isolated from human skin tissue (360-400 µm thick) of the anterior and posterior thigh areas. The receiving fluid consisted of 10 ml of 2% bovine serum albumin in normal saline and was stirred at 600 rpm. The cumulative amounts of these prodrugs and their parent drugs penetrating the skin versus time were determined by a specific high-performance liquid chromatography method. The results using a donor consisting of either a 30% solution of diethylaminoethyl 1-(p-chlorobenzoyl)-5-methoxy-2-methylindole 3-acetate.AcOH, diethylaminoethyl (Z)-5-fluoro-2-methyl-1-[(4-methylsulfinyl) phenylmethylene] - 1H-indene-3-acetate.AcOH, diethylaminoethyl 1-methyl-5-(4-methylbenzoyl)-1H-pyrrole-2-acetate.AcOH, diethylaminoethyl 5-(4-Chlorobenzoyl)-1,4-dimethyl-1H-pyrrole-2-acetate.AcOH, diethylaminoethyl 1,8-diethyl-1,3,4,9-tetrahydropyrano-[3,4-b]indole-1-acetate.AcOH, diethylaminoethyl 2-amino-3-benzoylbenzeneacetate.AcOH, diethylaminoethyl 2-amino-3-(4-bromo-benzoyl)benzeneacetate.AcOH, diethylaminoethyl 3-chloro-4-(2-propenyloxy)benzeneacetate.AcOH, diethylaminoethyl 2-(2,4-dichlorophenoxy)benzeneacetate.AcOH, diethylaminoethyl 1-(4-chlorobenzoyl-5-methoxy-2-methyl-1H-indole-3-acetoxyacetate.AcOH, diethylaminoethyl4-(4-chlorophenyl)-2-phenyl-5-thiazoleacetate.AcOH, or a 30% suspension of indomethacin, sulindac, tolmetin, zomepirac, etodolac, amfenac, bromofenac, alclofenac, fenclofenac, acemetacin, or fentiazac in 2mL of pH 7.4 phosphate buffer (0.2M) are shown in Figure 1 or Figure 2. Apparent flux values of 4.6, 4, 4.2, 5.1, 4.2, 3.8, 4.0, 3.6, 4.5, 3.8, 4.5, 0.04, 0.04, 0.04, 0.05, 0.04, 0.04, 0.04, 0.03, 0.04, 0.03, and 0.04 mg/cm²/h were calculated for diethylaminoethyl 1-(p-chlorobenzoyl)-5-methoxy-2-methylindole 3-acetate.AcOH, diethylaminoethyl (Z)-5-fluoro-2-methyl-l-[(4-methylsulfinyl) phenylmethylene] - 1H-indene-3-acetate.AcOH, diethylaminoethyl 1-methyl-5-(4-methylbenzoyl)-1H-pyrrole-2-acetate.AcOH, diethylaminoethyl 5-(4-Chlorobenzoyl)-1,4-dimethyl-1H-pyrrole-2-acetate.AcOH, diethylaminoethyl 1,8-diethyl-1,3,4,9-tetrahydropyrano-[3,4-b]indole-1-acetate.AcOH, diethylaminoethyl 2-amino-3-benzoylbenzeneacetate.AcOH, diethylaminoethyl 2-amino-3-(4-bromo-benzoyl)benzeneacetate.AcOH, diethylaminoethyl 3-chloro-4-(2-propenyloxy)benzeneacetate.AcOH, diethylaminoethyl 2-(2,4-dichlorophenoxy)benzeneacetate.AcOH, diethylaminoethyl 1-(4-chlorobenzoyl-5-methoxy-2-methyl-1H-indole-3-acetoxyacetate.AcOH, diethylaminoethyl4-(4-chlorophenyl)-2-phenyl-5-thiazoleacetate.AcOH, indomethacin, sulindac, tolmetin, zomepirac, etodolac, amfenac, bromofenac, alclofenac, fenclofenac, acemetacin, fentiazac, and lonazolac, respectively, to diffuse through human skin. The results suggest that the pro-drug, diethylaminoethyl1-(p-chlorobenzoyl)-5-methoxy-2-methylindole 3-acetate.AcOH, diethylaminoethyl (Z)-5-fluoro-2-methyl-1- [(4-methylsulfinyl) phenylmethylene]-1H-indene-3-acetate.AcOH, diethylaminoethyl 1-methyl-5-(4-methylbenzoyl)-1 H-pyrrole-2-acetate.AcOH, diethylaminoethyl 5-(4-Chlorobenzoyl)-1,4-dimethyl-1H-pyrrole-2-acetate.AcOH, or diethylaminoethyl 1,8-diethyl-1,3,4,9-tetrahydropyrano-[3,4-b]indole-1-acetate.AcOH, diethylaminoethyl 2-amino-3-benzoylbenzeneacetate.AcOH, diethylaminoethyl 2-amino-3-(4-bromo-benzoyl)benzeneacetate.AcOH, diethylaminoethyl 3-chloro-4-(2-propenyloxy)benzeneacetate.AcOH, diethylaminoethyl 2-(2,4-dichlorophenoxy)benzeneacetate.AcOH, diethylaminoethyl 1-(4-chlorobenzoyl-5-methoxy-2-methyl-1H-indole-3-acetoxyacetate.AcOH, or diethylaminoethyl 4-(4-chlorophenyl)-2-phenyl-5-thiazoleacetate.AcOH, diffuses through human skin ∼100 times faster than indomethacin, sulindac, tolmetin, zomepirac, etodolac, amfenac, bromofenac, alclofenac, fenclofenac, acemetacin, fentiazac, or lonazolac. The results suggest that the positive charge on the dialkyaminoethyl group has a very important role in the passage of the drug across the membrane and skin barrier.

The in vivo rates of penetration of diethylaminoethyl 1-(p-chlorobenzoyl)-5-methoxy-2-methylindole 3-acetate.AcOH, diethylaminoethyl (Z)-5-fluoro-2-methyl-1-[(4-methylsulfinyl) phenylmethylene] - 1 H-indene-3-acetate.AcOH, diethylaminoethyl 1-methyl-5-(4-methylbenzoyl)-1H-pyrrole-2-acetate.AcOH, diethylaminoethyl 5-(4-Chlorobenzoyl)-1,4-dimethyl-1H-pyrrole-2-acetate.AcOH, diethylaminoethyl 1,8-diethyl-1,3,4,9-tetrahydropyrano-[3,4-b]indole-1-acetate.AcOH, diethylaminoethyl 2-amino-3 -benzoylbenzeneacetate.AcOH, diethylaminoethyl 2-amino-3-(4-bromo-benzoyl)benzeneacetate.AcOH, diethylaminoethyl 3-chloro-4-(2-propenyloxy)benzeneacetate.AcOH, diethylaminoethyl 2-(2,4-dichlorophenoxy)benzeneacetate.AcOH, diethylaminoethyl 1-(4-chlorobenzoyl-5-methoxy-2-methyl-1H-indole-3-acetoxyacetate.AcOH, diethylaminoethyl4-(4-chlorophenyl)-2-phenyl-5-thiazoleacetate.AcOH, diethylaminoethyl 3-(4-chlorophenyl)-1-phenyl-1H-pyrazole-4-acetate.AcOH, indomethacin, sulindac, tolmetin, zomepirac, etodolac, amfenac, bromofenac, alclofenac, fenclofenac, acemetacin, fentiazac, or lonazolac through the skin of intact hairless mice were compared. The donor consisted of a 20% solution of diethylaminoethyl 1-(p-chlorobenzoyl)-5-methoxy-2-methylindole 3-acetate.AcOH, diethylaminoethyl (Z)-5-fluoro-2-methyl-1-[(4-methylsulfinyl) phenylmethylene] - 1 H-indene-3-acetate.AcOH, diethylaminoethyl 1-methyl-5-(4-methylbenzoyl)-1H-pyrrole-2-acetate.AcOH, diethylaminoethyl 5-(4-Chlorobenzoyl)-1,4-dimethyl-1H-pyrrole-2-acetate.AcOH, diethylaminoethyl 1,8-diethyl-1,3,4,9-tetrahydropyrano-[3,4-b]indole-1-acetate.AcOH, diethylaminoethyl 2-amino-3-benzoylbenzeneacetate.AcOH, diethylaminoethyl 2-amino-3-(4-bromo-benzoyl)benzeneacetate.AcOH, diethylaminoethyl 3-chloro-4-(2-propenyloxy)benzeneacetate.AcOH, diethylaminoethyl 2-(2,4-dichlorophenoxy)benzeneacetate.AcOH, diethylaminoethyl 1-(4-chlorobenzoyl-5-methoxy-2-methyl-1H-indole-3-acetoxyacetate.AcOH, diethylaminoethyl4-(4-chlorophenyl)-2-phenyl-5-thiazoleacetate.AcOH, indomethacin, sulindac, tolmetin, zomepirac, etodolac, amfenac, bromofenac, alclofenac, fenclofenac, acemetacin, fentiazac, or lonazolac in 1 mL of isopropanol applied to a 10 cm on the backs of the hairless mice. Plasma levels of indomethacin, sulindac, tolmetin, zomepirac, etodolac, amfenac, bromofenac, alclofenac, fenclofenac, acemetacin, fentiazac, or lonazolac were determined by a specific high-performance liquid chromatography method. The results (Figure 3, Figure 4, and Figure 5) show that the peak levels of diethylaminoethyl1-(p-chlorobenzoyl)-5-methoxy-2-methylindole 3-acetate.AcOH, diethylaminoethyl (Z)-5-fluoro-2-methyl-1-[(4-methylsulfinyl) phenylmethylene]-1 H-indene-3-acetate.AcOH, diethylaminoethyl 1-methyl-5-(4-methylbenzoyl)-1H-pyrrole-2-acetate.AcOH, diethylaminoethyl 5-(4-Chlorobenzoyl)-1,4-dimethyl-1H-pyrrole-2-acetate.AcOH, diethylaminoethyl 1,8-diethyl-1,3,4,9-tetrahydropyrano-[3,4-b]indole-1-acetate.AcOH, diethylaminoethyl 2-amino-3-benzoylbenzeneacetate.AcOH, diethylaminoethyl 2-amino-3-(4-bromo-benzoyl)benzeneacetate.AcOH, diethylaminoethyl 3-chloro-4-(2-propenyloxy)benzeneacetate.AcOH, diethylaminoethyl 2-(2,4-dichlorophenoxy)benzeneacetate.AcOH, diethylaminoethyl 1-(4-chlorobenzoyl-5-methoxy-2-methyl-1H-indole-3-acetoxyacetate.AcOH, diethylaminoethyl 4-(4-chlorophenyl)-2-phenyl-5-thiazoleacetate.AcOH, and diethylaminoethyl 3-(4-chlorophenyl)-1-phenyl-1H-pyrazole-4-acetate.AcOH were reached ∼50 minutes after application of the donor systems. It takes 2-4 hours for indomethacin, sulindac, tolmetin, zomepirac, etodolac, amfenac, bromofenac, alclofenac, fenclofenac, acemetacin, and fentiazac to reach their peak plasma level when they are taken orally. The peaks were ∼0.01 mg/ml for indomethacin, sulindac, tolmetin, zomepirac, etodolac, amfenac, bromofenac, alclofenac, fenclofenac, acemetacin, and fentiazac, lonazolac, bendazac, and ∼2 mg/ml for diethylaminoethyl 1-(p-chlorobenzoyl)-5-methoxy-2-methylindole 3-acetate.AcOH, diethylaminoethyl (Z)-5-fluoro-2-methyl-1-[(4-methylsulfinyl) phenylmethylene] - 1H-indene-3-acetate.AcOH, diethylaminoethyl 1-methyl-5-(4-methylbenzoyl)-1H-pyrrole-2-acetate.AcOH, diethylaminoethyl 5-(4-Chlorobenzoyl)-l,4-dimethyl-1H-pyrrole-2-acetate.AcOH, diethylatninoethyl 1,8-diethyl-1,3,4,9-tetrahydropyrano-[3,4-b]indole-1-acetate.AcOH, diethylaminoethyl 2-amino-3-benzoylbenzeneacetate.AcOH, diethylaminoethyl 2-amino-3-(4-bromo-benzoyl)benzeneacetate.AcOH, diethylaminoethyl 3-chloro-4-(2-propenyloxy)benzeneacetate.AcOH, diethylaminoethyl 2-(2,4-dichlorophenoxy)benzeneacetate.AcOH, diethylaminoethyl 1-(4-chlorobenzoyl-5-methoxy-2-methyl-1H-indole-3-acetoxyacetate.AcOH, diethylaminoethyl4-(4-chlorophenyl)-2-phenyl-5-thiazoleacetate.AcOH, diethylaminoethyl 3-(4-chlorophenyl)-1-phenyl-1H-pyrazole-4-acetate.AcOH, (approximately 200 times of difference). ∼2 mg/ml of indomethacin, sulindac, tolmetin, zomepirac, etodolac, amfenac, bromofenac, alclofenac, fenclofenac, acemetacin, or fentiazac in plasma is more than 20-100 times higher than plasma level for effective analgesia and effective anti-inflammatory activity. This is a very exciting result. It will be very easy and fast to deliver therapeutically effective plasma level of indomethacin, sulindac, tolmetin, zomepirac, etodolac, amfenac, bromofenac, alclofenac, fenclofenac, acemetacin, or fentiazac into the host by these prodrugs. These results suggest that the pro-drugs can be administered not only orally, but also transdermally.

To check the gastroduodenal bleeding caused by drugs, rats were orally administered with 50 mg/kg of diethylaminoethyl 1-(p-chlorobenzoyl)-5-methoxy-2-methylindole 3-acetate.AcOH, diethylaminoethyl (Z)-5-fluoro-2-methyl-1-[(4-methylsulfinyl) phenylmethylene] - 1 H-indene-3-acetate.AcOH, diethylaminoethyl 1-methyl-5-(4-methylbenzoyl)-1H-pyrrole-2-acetate.AcOH, diethylaminoethyl 5-(4-Chlorobenzoyl)-1,4-dimethyl-1H-pyrrole-2-acetate.AcOH, diethylaminoethyl 1,8-diethyl-1,3,4,9-tetrahydropyrano-[3,4-b]indole-1-acetate.AcOH, diethylaminoethyl 2-amino-3-benzoylbenzeneacetate.AcOH, diethylaminoethyl 2-amino-3-(4-bromo-benzoyl)benzeneacetate.AcOH, diethylaminoethyl 3-chloro-4-(2-propenyloxy)benzeneacetate.AcOH, diethylaminoethyl 2-(2,4-dichlorophenoxy)benzeneacetate.AcOH, diethylaminoethyl 1-(4-chlorobenzoyl-5-methoxy-2-methyl-1H-indole-3-acetoxyacetate.AcOH, diethylaminoethyl4-(4-chlorophenyl)-2-phenyl-5-thiazoleacetate.AcOH, diethylaminoethyl 3-(4-chlorophenyl)-1-phenyl-1H-pyrazole-4-acetate.AcOH, indomethacin, sulindac, tolmetin, zomepirac, etodolac, amfenac, bromofenac, alclofenac, fenclofenac, acemetacin, fentiazac, or lonazolac per day for 21 days. We found an average of 3-4 mg of fecal blood per gram of feces in the indomethacin, sulindac, tolmetin, zomepirac, etodolac, amfenac, bromofenac, alclofenac, fenclofenac, acemetacin, fentiazac, or lonazolac groups, but none in the diethylaminoethyl 1-(p-chlorobenzoyl)-5-methoxy-2-methylindole 3-acetate.AcOH, diethylaminoethyl (Z)-5-fluoro-2-methyl-1-[(4-methylsulfinyl) phenylmethylene] - 1H-indene-3-acetate.AcOH, diethylaminoethyl 1-methyl-5-(4-methylbenzoyl)-1H-pyrrole-2-acctate.AcOH, diethylaminoethyl 5-(4-Chlorobenzoyl)-1,4-dimethyl-1H-pyrrole-2-acetate.AcOH, diethylaminoethyl 1,8-diethyl-1,3,4,9-tetrahydropyrano-[3,4-b]indole-1-acetate.AcOH, diethylaminoethyl 2-amino-3-benzoylbenzeneacetate.AcOH, diethylaminoethyl 2-amino-3-(4-bromo-benzoyl)benzeneacetate.AcOH, diethylaminoethyl 3-chloro-4-(2-propenyloxy)benzeneacetate.AcOH, diethylaminoethyl 2-(2,4-dichlorophenoxy)benzeneacetate.AcOH, diethylaminoethyl 1-(4-chlorobenzoyl-5-methoxy-2-methyl-1H-indole-3-acetoxyacetate.AcOH, diethylaminoethyl4-(4-chlorophenyl)-2-phenyl-5-thiazoleacetate.AcOH, or

diethylaminoethyl 3-(4-chlorophenyl)-1-phenyl-1H-pyrazole-4-acetate.AcOH groups. The acute toxicity of the prodrugs was investigated. The oral LD₅₀ in rats are: 0.1 g/kg, 0.5 g/kg, 0.6g/kg, 0.2 g/kg, 0.6 g/kg, 1.0 g/kg, 1.6 g/kg, 3.0 g/kg, 0.2 g/kg, 1.2 g/kg, and 1.2 g/kg for diethylaminoethyl1-(p-chlorobenzoyl)-5-methoxy-2-methylindole 3-acetate.AcOH, diethylaminoethyl (Z)-5-fluoro-2-methyl-1-[(4-methylsulfinyl) phenylmethylene]-1H-indene-3-acetate.AcOH, diethylaminoethyl 1-methyl-5-(4-methylbenzoyl)-1H-pyrrole-2-acetate.AcOH, diethylaminoethyl 5-(4-Chlorobenzoyl)-1,4-dimethyl-1H-pyrrole-2-acetate.AcOH, diethylaminoethyl 1,8-diethyl-1,3,4,9-tetrahydropyrano-[3,4-b]indole-1-acetate.AcOH, diethylaminoethyl 2-amino-3-benzoylbenzeneacetate.AcOH, diethylaminoethyl 2-amino-3-(4-bromo-benzoyl)benzeneacetate.AcOH, diethylaminoethyl 3-chloro-4-(2-propenyloxy)benzeneacetate.AcOH, diethylaminoethyl 2-(2,4-dichlorophenoxy)benzeneacetate.AcOH, diethylaminoethyl 1-(4-chlorobenzoyl-5-methoxy-2-methyl-1H-indole-3-acetoxyacetate.AcOH, diethylaminoethyl 4-(4-chlorophenyl)-2-phenyl-5-thiazoleacetate.AcOH, and diethylaminoethyl 3-(4-chlorophenyl)-1-phenyl-1H-pyrazole-4-acetate.AcOH, respectively. The results show that the prodrugs are less toxic than indomethacin (LD =0.013g/kg), sulindac (LD₅₀ =0.26 g/kg), tolmetin (LD₅₀ =0.35 g/kg), zomepirac (LD₅₀ =0.027 g/kg), etodolac (LD₅₀ =0.46 g/kg), amfenac (LD₅₀ =0.31 g/kg), bromofenac, alclofenac (LD ₅₀ =1.05 g/kg), fenclofenac (LD₅₀ =2.28 g/kg), acemetacin (LD₅₀ = 0.024 g/kg), fentiazac (LD₅₀=0.7 g/kg), or lonazolac (LD₅₀ =1.0 g/kg).

Tolmetin, zomepirac, etodolac, amfenac, bromofenac, alclofenac, fenclofenac, indomethacin, sulindac, acemetacin, fentiazac, lonazolac, bendazac, 6MNA, ibufenac, and related compounds have demonstrated anti-inflammatory, analgesic, antipyretic, and antirheumatic activities. A good prodrug should go back to its parent drug in plasma. The diethylaminoethyl ester group of these prodrugs can be rapidly cleaved by the enzymes in human plasma in vitro and more than 90% of the pro-drugs are changed back to their parent drugs. Due to the fact that the pro-drugs have a much better absorption rate, the prodrugs will have a higher efficacy than their parent drugs at the same dosage. The analgetic, antipyretic and anti-inflammatory activities of these prodrugs were tested relative to tolmetin, zomepirac, etodolac, amfenac, indomethacin, sulindac, bromofenac, alclofenac, fenclofenac, acemetacin, fentiazac, or lonazolac .

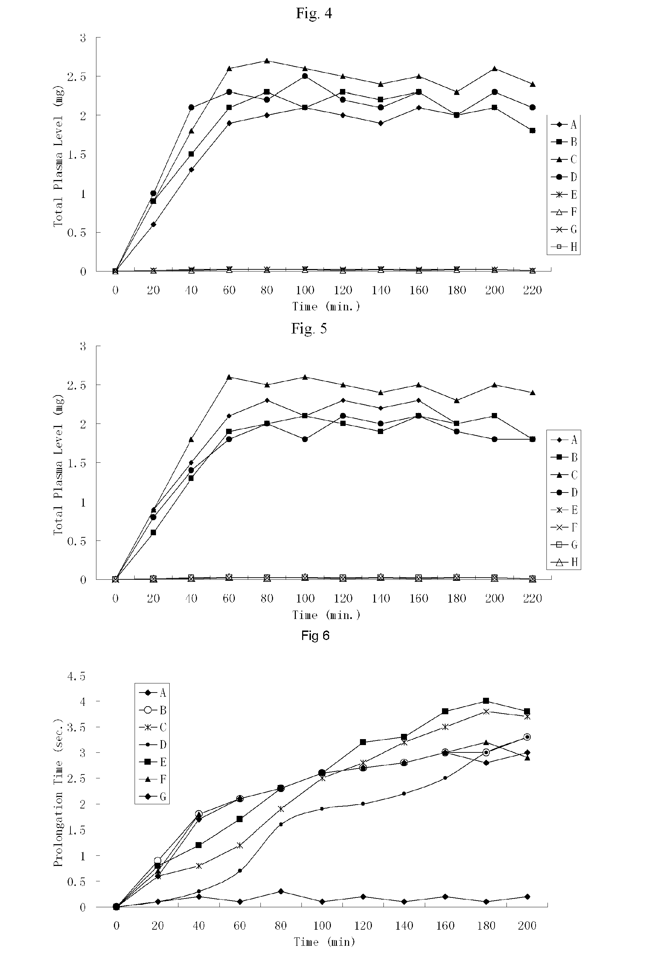

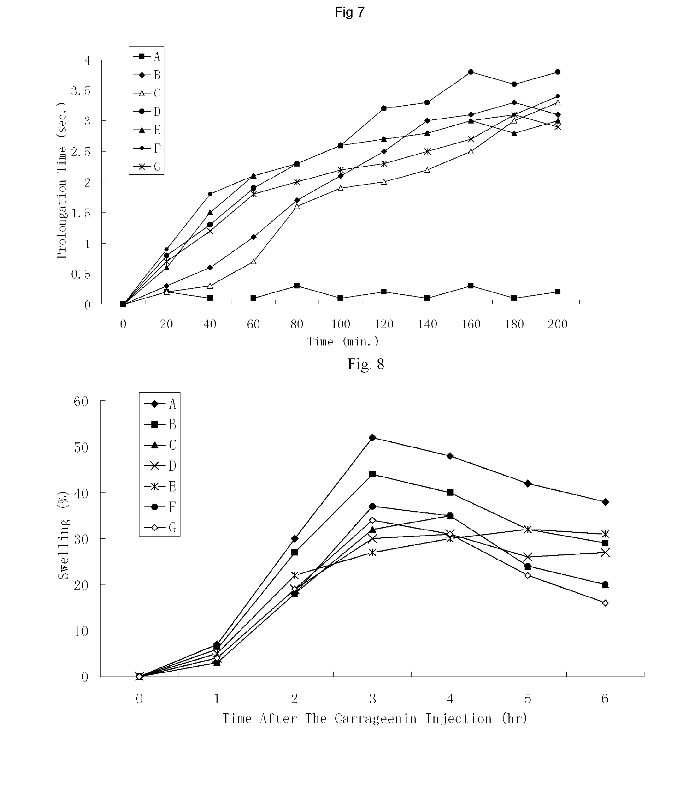

Analgetic activity: The prolongation time of pain threshold of a mouse tail was determined in accordance with the D'Amour-Smith Method (J. Pharmacal. Exp. Ther., 72,74(1941)). After 50 mg/kg of these prodrugs were administered transdermally, the tails of mice were exposed to heat and the prolongation time of pain threshold was determined. The results obtained are shown in Figure 6 and Figure 7. Diethylaminoethyl 1-(p-chlorobenzoyl)-5-methoxy-2-methylindole 3-acetate.AcOH, diethylaminoethyl (Z)-5-fluoro-2-methyl-1-[(4-methylsulfinyl) phenylmethylene] - 1H-indene-3-acetate.AcOH, diethylaminoethyl 1-methyl-5-(4-methylbenzoyl)-1H-pyrrole-2-acetate.AcOH, diethylaminoethyl 5-(4-Chlorobenzoyl)-1,4-dimethyl-1H-pyrrole-2-acetate.AcOH, diethylaminoethyl 1,8-diethyl-1,3,4,9-tetrahydropyrano-[3,4-b]indole-1-acetate.AcOH, diethylaminoethyl 2-amino-3-benzoylbenzeneacetate.AcOH, diethylaminoethyl 2-amino-3-(4-bromo-benzoyl)benzeneacetate.AcOH, diethylaminoethyl 3-chloro-4-(2-propenyloxy)benzeneacetate.AcOH, diethylaminoethyl 2-(2,4-dichlorophenoxy)benzeneacetate.AcOH, diethylaminoethyl 1-(4-chlorobenzoyl-5-methoxy-2-methyl-1H-indole-3-acetoxyacetate.AcOH, diethylaminoethyl 4-(4-chlorophenyl)-2-phenyl-5-thiazoleacetate.AcOH, and diethylaminoethyl 3-(4-chlorophenyl)-1-phenyl-1H-pyrazole-4-acetate.AcOH have shown analgesic activity nicely.

The number of writhings that occurred when mice were administered an acetic acid solution intraperitoneally were counted, and the rate of inhibition relative to the control group was calculated. Diethylaminoethyl 1-(p-chlorobenzoyl)-5-methoxy-2-methylindole 3-acetate.AcOH (100 mg/kg, B), diethylaminoethyl (Z)-5-fluoro-2-methyl-1-[(4-methylsulfinyl) phenylmethylene] - 1H-indene-3-acetate.AcOH (100 mg/kg, C), diethylaminoethyl 1-methyl-5-(4-methylbenzoyl)-1H-pyrrole-2-acetate.AcOH (100 mg/kg, D), diethylaminoethyl 5-(4-Chlorobenzoyl)-1,4-dimethyl-1H-pyrrole-2-acetate.AcOH (100 mg/ kg, E), diethylaminoethyl1,8-diethyl-1,3,4,9-tetrahydropyrano-[3,4-b] indole-1-acetate.AcOH (100 mg/kg, F), diethylaminoethyl 2-amino-3-benzoylbenzeneacetate.AcOH (100 mg/kg, G), diethylaminoethyl 2-amino-3-(4-bromo-benzoyl)benzeneacetate.AcOH (100 mg/kg, H), diethylaminoethyl3-chloro-4-(2-propenyloxy)benzeneacetate.AcOH (100 mg/kg, 1), diethylaminoethyl2-(2,4-dichlorophenoxy)benzeneacetate.AcOH (100 mg/kg, J), diethylaminoethyl 1-(4-chlorobenzoyl-5-methoxy-2-methyl-lH-indole-3-acetoxyacetate.AcOH (100 mg/ kg, K), diethylaminoethyl 4-(4-chlorophenyl)-2-phenyl-5-thiazolcacetate.AcOH (100 mg/kg, L), or diethylaminoethyl 3-(4-chlorophenyl)-1-phenyl-1H-pyrazole-4-acetate.AcOH (100 mg/kg, M) were administered transdermally t o the mice 30 minutes before the acetic acid solution was ad- ministered. The A group is the control group. The results are shown in Table 1.

**Table 1. The rate of writhings inhibition by and ketoprofen and related compounds.**

| Group | Dose (mg/kg) | No. of Writhings | % |
|---|---|---|---|
| A | 0 | 35.0 | - |
| B | 100 | 15.6 | 55 |
| C | 100 | 14.2 | 59 |
| D | 100 | 17.1 | 51 |
| E | 100 | 15.6 | 55 |
| F | 100 | 14.0 | 60 |
| G | 100 | 13.8 | 61 |
| H | 100 | 13.2 | 62 |
| I | 100 | 15.7 | 55 |
| J | 100 | 14.2 | 59 |
| K | 100 | 15.6 | 55 |
| L | 100 | 16.1 | 54 |
| M | 100 | 15.2 | 57 |

The results show that the prodrugs demonstrated exceptional analgetic activity.

Antipyretic activity: Rats received a sterilized *E. coli* suspension as a pyrogen.

The control group is group A. 2 hours later, diethylaminoethyl 1-(p-chlorobenzoyl)-5-methoxy-2-methylindole 3-acetate.AcOH (100 mg/kg, B), diethylaminoethyl (Z)-5-fluoro-2-methyl-1-[(4-methylsulfinyl) phenylmethylene] - 1H-indene-3-acetate.AcOH (100 mg/kg, C), diethylaminoethyl 1-methyl-5-(4-methylbenzoyl)-1H-pyrrole-2-acetate.AcOH (100 mg/kg, D), diethylaminoethyl 5-(4-Chlorobenzoyl)-1,4-dimethyl-1H-pyrrole-2-acetate.AcOH (100 mg/ kg, E), diethylaminoethyl1,8-diethyl-1,3,4,9-tetrahydropyrano-[3,4-b] indole-1-acetate.AcOH (100 mg/kg, F), diethylaminoethyl 2-amino-3-benzoylbenzeneacetate.AcOH (100 mg/kg, G), diethylaminoethyl 2-amino-3-(4-bromo-benzoyl)benzeneacetate.AcOH (100 mg/kg, H), diethylaminoethyl3-chloro-4-(2-propenyloxy)benzeneacetate.AcOH (100 mg/kg, 1), diethylaminoethyl2-(2,4-dichlorophenoxy)benzeneacetate.AcOH (100 mg/kg, J), diethylaminoethyl 1-(4-chlorobenzoyl-5-methoxy-2-methyl-1H-indole-3-acetoxyacetate.AcOH (100 mg/ kg, K), diethylaminoethyl4-(4-chlorophenyl)-2-phenyl-5-thiazoleacetate.AcOH (100 mg/kg, L), or diethylaminoethyl 3-(4-chlorophenyl)-1-phenyl-1H-pyrazole-4-acetate.AcOH (100 mg/kg, M) was administered transdermally. The body temperature of rats was taken at 90 min. intervals before and after the administration of the test compounds. The results are shown in Table 2.

**Table 2. Antipyretic Activity of ketoprofen and related compounds.**

| Compound | t=O min. | t=90 min. | t=180 min. | t=270 min. |
|---|---|---|---|---|
| A (Control group) | 37.33±0.05 | 37.26±0.07 | 37.32±0.05 | 37.34±0.08 |
| B (100 mg/kg) | 37.35±0.06 | 36.91±0.05 | 36.85±0.08 | 36.79±0.07 |
| C (100mg/kg) | 37.28±0.06 | 36.65±0.05 | 36.62±0.08 | 36.58±0.07 |
| D (100mg/kg) | 37.27±0.06 | 36.71±0.05 | 36.65±0.08 | 36.59±0.07 |
| E (100mg/kg) | 37.21±0.07 | 36.82±0.06 | 36.70±0.05 | 36.70±0.08 |
| F (100mg/kg) | 37.23±0.06 | 36.65±0.06 | 36.58±0.08 | 36.60±0.07 |
| G (100mg/kg) | 37.22±0.06 | 36.65±0.05 | 36.62±0.08 | 36.58±0.07 |
| H (100mg/kg) | 37.25±0.06 | 36.71±0.05 | 36.65±0.08 | 36.64±0.07 |
| I (100mg/kg) | 37.23±0.07 | 36.80±0.06 | 36.70±0.05 | 36.67±0.08 |
| J (100mg/kg) | 37.22±0.06 | 36.65±0.06 | 36.58±0.08 | 36.56±0.07 |
| K (100mg/kg) | 37.21±0.06 | 36.75±0.05 | 36.62±0.08 | 36.58±0.07 |
| L (100mg/kg) | 37.23±0.06 | 36.81±0.05 | 36.75±0.08 | 36.71±0.07 |
| M (100mg/kg) | 37.22±0.07 | 36.82±0.06 | 36.80±0.05 | 36.77±0.08 |

The results show that the prodrugs demonstrated strong antipyretic activity at 100 mg/kg dose.

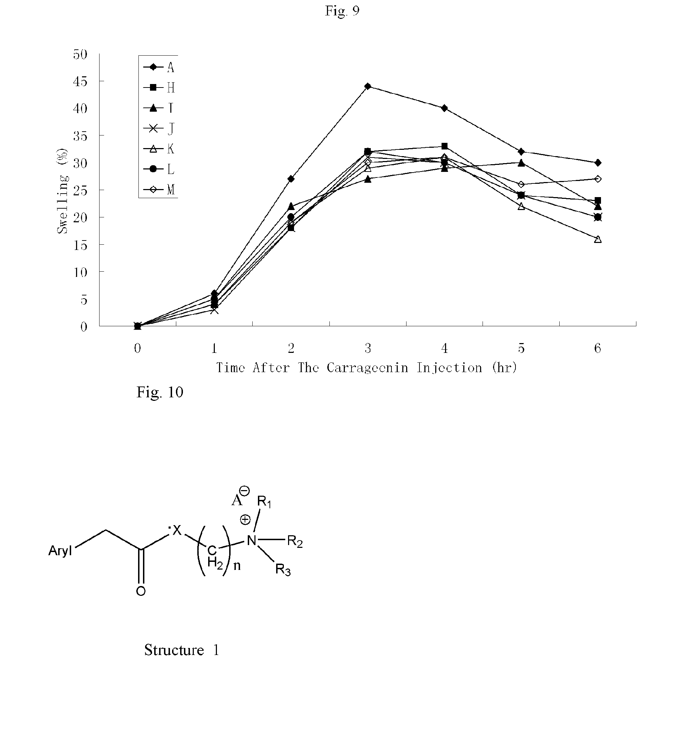

Anti-inflammatory activity: 100 mg/kg of diethylaminoethyl 1-(p-chlorobenzoyl)-5-methoxy-2-methylindole 3-acetate.AcOH (B), diethylaminoethyl (Z)-5-fluoro-2-methyl-1-[(4-methylsulfinyl) phenylmethylene] - 1H-indene-3-acetate.AcOH (C), diethylaminoethyl 1-methyl-5-(4-methylbenzoyl)-1H-pyrrole-2-acetate.AcOH (D), diethylaminoethyl 5-(4-Chlorobenzoyl)-1,4-dimethyl-1H-pyrrole-2-acetate.AcOH (E), diethylaminoethyl 1,8-diethyl-1,3,4,9-tetrahydropyrano-[3,4-b]indole-1-acetate.AcOH (F), diethylaminoethyl 2-amino-3-benzoylbenzeneacetate.AcOH (G), diethylaminoethyl 2-amino-3-(4-bromo-benzoyl)benzeneacetate.AcOH (H), diethylaminoethyl 3-chloro-4-(2-propenyloxy)benzeneacetate.AcOH (I), diethylaminoethyl 2-(2,4-dichlorophenoxy)benzeneacetate.AcOH (J), diethylaminoethyl 1-(4-chlorobenzoyl-5-methoxy-2-methyl-1H-indole-3-acetoxyacetate.AcOH (100 mg/ kg, K), diethylaminoethyl4-(4-chlorophenyl)-2-phenyl-5-thiazoleacetate.AcOH (L), or diethylaminoethyl 3-(4-chlorophenyl)-1-phenyl-1H-pyrazole-4-acetate.AcOH (M) was administered transdermally. Group A is the control group. 60 minutes later, a carrageenin solution was administered subcutaneously to the foot pads of the rats. The volume of the hind paw was measured every hour after the administration of carrageenin, and the rate of increase in the volume of the paw was calculated and designated as the rate of swelling (%). The results obtained are shown in Figure 8 and Figure 9. The results show that these prodrugs after transdermal administration demonstrated good anti-inflammatory activity.

It is also known that a high oral dose of some NSAIAs has an anti- reactive-antiasthmatic activity by inhibition of the cyclooxygenase activity. Due to their very high membrane penetration rate, these prodrugs can be used in a method for treating asthma by spraying into the mouth or the nose of the host. They can also be used in a method for treating acne due to their anti-inflammatory properties and very high skin penetration rate.

The present invention relates to pharmaceutical preparations comprising aforementioned prodrugs in addition to customary auxiliaries and excipients, e.g. in the form of tablets, capsules or solutions for oral administration and in the form of solutions, lotion, ointment, emulsion or gel for transdermal administration. The presently disclosed prodrugs can be combined with vitamins such as A, B, C or E or beta-carotene, or other pharmaceuticals, such as folic acid, etc., for use in a method for treating any NSAIAs-treatable conditions in humans or animals.

Transdermal therapeutic application systems comprising at least one presently disclosed prodrug as an active ingredient can be used in a method for treating any NSAIAs-treatable conditions in humans or animals. These systems can be a bandage or a patch comprising one active substance-containing matrix layer and an impermeable backing layer. The most preferable system is an active substance reservoir, which has a permeable bottom facing the skin. By controlling the rate of release, this system enables NSAIAs to reach constantly optimal therapeutic blood levels to increase effectiveness and reduce the side effects of NSAIAs. These systems can be worn on the wrist, ankle, arm, leg, or any part of the body.

The presently disclosed prodrugs can be prepared from acid halides or mixed anhydrides of tolmetin, zomepirac, etodolac, amfenac, bromofenac, alclofenac, fenclofenac, acemetacin, fentiazac, indomethacin, sulindac, lonazolac, bendazac, 6MNA or ibufenac with compounds of the general Structure 3. 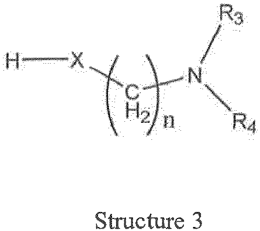 In structure 3, R₃ represents ethyl; R₄ represents ethyl; X represents O; and n=2.

The presently disclosed prodrugs can be prepared from tolmetin, zomepirac, etodolac, amfenac, bromofenac, alclofenac, fenclofenac, acemetacin, indomethacin, sulindac, fentiazac, lonazolac, bendazac, 6MNA, ibufenac, and related compounds, by reaction with compounds of the general Structure 3 using coupling reagents, such as N,N'-Dicyclohexylcarbodiimide, N, N'-Diisopropylcarbodiimide, O-(Benzotriazol-1-yl)-N,N,N',N'-tetramethyluronium tetrafluoroborate, O-(Benzotriazol-1-yl)-N,N,N',N'-tetramethyluronium hexafluorophosphate, Benzotriazol-1-yl-oxy-tris (dimethylamino)phosphonium hexafluorophosphate, et al.

The presently disclosed prodrugs can be prepared from metal salts, organic base salts or immobilized base salts of tolmetin, zomepirac, etodolac, amfenac, bromofenac, alclofenac, fenclofenac, acemetacin, indomethacin, sulindac, fentiazac, lonazolac, bendazac, 6MNA, ibufenac, and related compounds, by reaction with compounds of the general Structure 4. 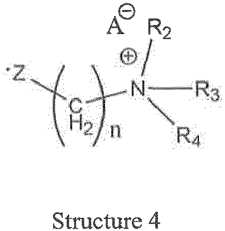 In structure 4, R2 represents H; R3 represents ethyl; R4 represents ethyl; Z represents halogen, or p-toluenesulphonyl, A⁻ represents any negative ions; and n=2.

### Advantageous Effects

These pro-drugs of tolmetin, zomepirac, etodolac, amfenac, bromofenac, alclofenac, fenclofenac, acemetacin, indomethacin, sulindac, fentiazac, lonazolac, bendazac, 6MNA, ibufenac, and related compounds have a lipophilic portion and a hydrophilic portion (the amine groups that exist in the protonated form at physiological pH). The positively charged amino groups of these pro-drugs have two major advantages. Firstly, they largely increase the solubility of the drugs; when these new prodrugs are administered orally in a dosage form such as a tablet, capsule, solution, or suspension, they will dissolve in gastric juice immediately. Secondly, the positive charge on the amino groups of these pro-drugs will bond to the negative charge on the phosphate head group of the membrane. Thus, the local concentration outside of the membrane will be very high and will facilitate the passage of these pro-drugs from a region of high concentration to a region of low concentration. When these pro-drugs enter the membrane, the hydrophilic part will push the pro-drugs into the cytosol, a semi-liquid concentrated aqueous solution or suspension. Due to the short stay in the GI tract, the pro-drugs will not cause gastric mucosal cell damage. Experiment results show that more than 90% of the pro-drugs were changed back to their parent drugs. The prodrugs have a much better absorption rate, and thus the pro-drugs will have a higher efficacy than tolmetin, zomepirac, etodolac, amfenac, bromofenac, alclofenac, fenclofenac, acemetacin, indomethacin, sulindac, fentiazac, lonazolac, bendazac, 6MNA, ibufenac, and related compounds at the same dosage. The experiment results suggest that the pro-drugs, i. e. diethylaminoethyl 1-(p-chlorobenzoyl)-5-methoxy-2-methylindole 3-acetate.AcOH, diethylaminoethyl (Z)-5-fluoro-2-methyl-1-[(4-methylsulfinyl) phenylmethylene] - 1H-indene-3-acetate.AcOH, diethylaminoethyl 1-methyl-5-(4-methylbenzoyl)-1H-pyrrole-2-acetate.AcOH, diethylaminoethyl 5-(4-Chlorobenzoyl)-1,4-dimethyl-1H-pyrrole-2-acetate.AcOH, diethylaminoethyl 1,8-diethyl-1,3,4,9-tetrahydropyrano-[3,4-b]indole-1-acetate.AcOH, diethylaminoethyl 2-amino-3-benzoylbenzeneacetate.AcOH, diethylaminoethyl 2-amino-3-(4-bromo-benzoyl)benzeneacetate.AcOH, diethylaminoethyl 3-chloro-4-(2-propenyloxy)benzeneacetate.AcOH, diethylaminoethyl 2-(2,4-dichlorophenoxy)benzeneacetate.AcOH, diethylaminoethyl 1-(4-chlorobenzoyl-5-methoxy-2-methyl-1H-indole-3-acetoxyacetate.AcOH, diethylaminoethyl4-(4-chlorophenyl)-2-phenyl-5-thiazoleacetate.AcOH, diethylaminoethyl 4-(4-chlorophenyl)-2-phenyl-5-thiazoleacetate.AcOH, and diethylaminoethyl 3-(4-chlorophenyl)-1-phenyl-1H-pyrazole-4-acetate.AcOH diffuse through human skin ∼100 times faster than tolmetin, zomepirac, etodolac, amfenac, bromofenac, alclofenac, fenclofenac, acemetacin, indomethacin, sulindac, fentiazac, lonazolac, bendazac, 6MNA, ibufenac, and related compounds. It takes 2-4 hours for tolmetin, zomepirac, etodolac, amfenac, bromofenac, alclofenac, fenclofenac, acemetacin, indomethacin, sulindac, fentiazac, lonazolac, bendazac, 6MNA, ibufenac, and related compounds to reach the peak plasma level when they are taken orally, but these prodrugs only take about 40-50 minutes to reach the peak plasma level. The most exciting result is that the pro-drugs can be administered not only orally, but also transdermally and should not have most of the side effects of tolmetin, zomepirac, etodolac, amfenac, bromofenac, alclofenac, fenclofenac, acemetacin, indomethacin, sulindac, fentiazac, lonazolac, bendazac, and related compounds, most notably GI disturbances such as dyspepsia, gastroduodenal bleeding, gastric ulcerations, and gastritis. Another great benefit of transdermal administration of these pro-drugs is that administering medication, especially to children, will be much easier.

### Description of Drawings

Figure 1: Cumulative amounts of diethylaminoethyl 1-(p-chlorobenzoyl)-5-methoxy-2-methylindole 3-acetate.AcOH (A, 30% solution), diethylaminoethyl (Z)-5-fluoro-2-methyl-1-[(4-methylsulfinyl) phenylmethylene] - 1H-indene-3-acetate.AcOH (B, 30% solution), diethylaminoethyl 1-methyl-5-(4-methylbenzoyl)-1H-pyrrole-2-acetate.AcOH (C, 30% solution), diethylaminoethyl 5-(4-Chlorobenzoyl)-1,4-dimethyl-1H-pyrrole-2-acetate.AcOH (D, 30% solution), diethylaminoethyl1,8-diethyl-1,3,4,9-tetrahydropyrano-[3,4-b] indole-1-acetate.AcOH (E, 30% solution), indomethacin (F, 30% suspension) , sulindac (G, 30% suspension), tolmetin (H, 30% suspension), zomepirac (I, 30% suspension), or etodolac (J, 30% suspension), crossing isolated human skin tissue in Franz cells (n=5). In each case, the vehicle was pH 7.4 phosphate buffer (0.2 M).
Figure 2: Cumulative amounts of diethylaminoethyl 2-amino-3-benzoylbenzeneacetate.AcOH (A, 30% solution), diethylaminoethyl 2-amino-3-(4-bromo-benzoyl)benzeneacetate.AcOH (B, 30% solution), diethylaminoethyl 3-chloro-4-(2-propenyloxy)benzeneacetate.AcOH (C, 30% solution), diethylaminoethyl2-(2,4-dichlorophenoxy) benzeneacetate.AcOH (D, 30% solution), diethylaminoethyl 1-(4-chlorobenzoyl-5-methoxy-2-methyl-1H-indole-3-acetoxyacetate.AcOH (E, 30% solution), diethylaminoethyl4-(4-chlorophenyl)-2-phenyl-5-thiazoleacetate.AcOH (F, 30% solution), amfenac (G, 30% suspension), bromofenac (H, 30% suspension), alclofenac (I, 30% suspension), fenclofenac (J, 30% suspension), acemetacin (K, 30% suspension), or fentiazac (L, 30% suspension), crossing isolated human skin tissue in Franz cells (n=5). In each case, the vehicle was pH 7.4 phosphate buffer (0.2 M).
Figure 3: Total plasma levels of indomethacin, sulindac, tolmetin, or zomepirac after topical application of 1 ml of a 20% solution of diethylaminoethyl 1-(p-chlorobenzoyl)-5-methoxy-2-methylindole 3-acetate.AcOH (A), diethylaminoethyl (Z)-5-fluoro-2-methyl-1-[(4-methylsulfinyl) phenylmethylene] - 1H-indene-3-acetate.AcOH (B), diethylaminoethyl 1-methyl-5-(4-methylbenzoyl)-1H-pyrrole-2-acetate.AcOH (C), diethylaminoethyl 5-(4-Chlorobenzoyl)-1,4-dimethyl-1H-pyrrole-2-acetate.AcOH (D), indomethacin (E), sulindac (F), tolmetin, (G), or zomepirac (H) in isopropanol to the backs of hairless mice (n=5).
Figure 4: Total plasma levels of etodolac, amfenac, bromofenac, or alclofenac after topical application of 1 ml of 20% solution of diethylaminoethyl1,8-diethyl-1,3,4,9-tetrahydropyrano-[3,4-b] indole-1-acetate.AcOH (A), diethylaminoethyl 2-amino-3-benzoylbenzeneacetate.AcOH (B), diethylaminoethyl 2-amino-3-(4-bromo-benzoyl)benzeneacetate.AcOH (C), diethylaminoethyl 3-chloro-4-(2-propenyloxy) benzeneacetate.AcOH (D), etodolac (E), amfenac (F), bromofenac (G), or alclofenac (H) in isopropanol to the back of hairless mice (n=5).
Figure 5: Total plasma levels of fenclofenac, acemetacin, fentiazac, or lonazolac after topical application of 1 ml of 20% solution of diethylaminoethyl 2-(2,4-dichlorophenoxy)benzeneacetate.AcOH (A), diethylaminoethyl 1-(4-chlorobenzoyl-5-methoxy-2-methyl-1H-indole-3-acetoxyacetate.AcOH (B), diethylaminoethyl 4-(4-chlorophenyl)-2-phenyl-5-thiazoleacetate.AcOH (C), diethylaminoethyl 3-(4-chlorophenyl)-1 -phenyl-1 H-pyrazole-4-acetate.AcOH (D), fenclofenac (E), acemetacin (F), fentiazac (G), or lonazolac )H) in isopropanol to the back of hairless mice (n=5).
Figure 6: The prolongation time of the pain threshold of mouse tails after 50mg/kg of diethylaminoethyl 1-(p-chlorobenzoyl)-5-methoxy-2-methylindole 3-acetate.AcOH (B), diethylaminoethyl (Z)-5-fluoro-2-methyl-1-[(4-methylsulfinyl) phenylmethylene] - 1H-indene-3-acetate.AcOH (C), diethylaminoethyl 1-methyl-5-(4-methylbenzoyl)-1H-pyrrole-2-acetate.AcOH (D), diethylaminoethyl 5-(4-Chlorobenzoyl)-1,4-dimethyl-1H-pyrrole-2-acetate.AcOH (E), diethylaminoethyl 1,8-diethyl-1,3,4,9-tetrahydropyrano-[3,4-b]indole-1-acetate.AcOH (F), or diethy- laminoethyl 2-amino-3-benzoylbenzeneacetate.AcOH (G) was administered transdermally. Group A is the control group.
Figure 7: The prolongation time of the pain threshold of mouse tails after 50mg/kg of diethylaminoethyl 2-amino-3-(4-bromo-benzoyl)benzeneacetate.AcOH (B), diethylaminoethyl 3-chloro-4-(2-propenyloxy)benzeneacetate.AcOH (C), diethylaminoethyl 2-(2,4-dichlorophenoxy)benzeneacetate.AcOH (D), diethylaminoethyl 1-(4-chlorobenzoyl-5-methoxy-2-methyl-1H-indole-3-acetoxyacetate.AcOH (E), diethylaminoethyl4-(4-chlorophenyl)-2-phenyl-5-thiazoleacetate.AcOH (F), diethylaminoethyl 3-(4-chlorophenyl)-1-phenyl-1H-pyrazole-4-acetate.AcOH (G). were administered transdermally. Group A is the control group.
Figure 8. The rate of swelling (%) after a carrageenin injection. 1 hour before the carrageenin injection, diethylaminoethyl 1-(p-chlorobenzoyl)-5-methoxy-2-methylindole 3-acetate.AcOH (100 mg/kg, B), diethylaminoethyl (Z)-5-fluoro-2-methyl-1-[(4-methylsulfinyl) phenylmethylene] - 1H-indene-3-acetate.AcOH (100 mg/kg, C), diethylaminoethyl 1-methyl-5-(4-methylbenzoyl)-1H-pyrrole-2-acetate.AcOH (100 mg/kg, D), diethylaminoethyl 5-(4-Chlorobenzoyl)-1,4-dimethyl-1H-pyrrole-2-acetate.AcOH (100 mg/ kg, E), diethylaminoethyl1,8-diethyl-1,3,4,9-tetrahydropyrano-[3,4-b] indole-1-acetate.AcOH (100 mg/kg, F), or diethylaminoethyl 2-amino-3-benzoylbenzeneacetate.AcOH (100 mg/kg, G) was administered transdermally. Group A is the control group.
Figure 9. The rate of swelling (%) after a carrageenin injection. 1 hour before the carrageenin injection, diethylaminoethyl 2-amino-3-(4-bromo-benzoyl)benzeneacetate.AcOH (100 mg/kg, H), diethylaminoethyl3-chloro-4-(2-propenyloxy)benzeneacetate.AcOH (100 mg/kg, 1), diethylaminoethyl2-(2,4-dichlorophenoxy)benzeneacetate.AcOH (100 mg/kg, J), diethylaminoethyl 1-(4-chlorobenzoyl-5-methoxy-2-methyl-1H-indole-3-acetoxyacetate.AcOH (100 mg/ kg, K), diethylaminoethyl4-(4-chlorophenyl)-2-phenyl-5-thiazoleacetate.AcOH (100 mg/kg, L), or diethylaminoethyl 3-(4-chlorophenyl)-1-phenyl-1H-pyrazole-4-acetate.AcOH (100 mg/kg, M) was administered transdermally. Group A is the control group.

### Examples

### Preparation of diethylaminoethyl

### (Z)-5-fluoro-2-methyl-1-[(4-methylsulfinyl) phenylmethylenel-1H-indene-3-acetate.AcOH

11.7 g (0.1 mol) of diethylaminoethanol was dissolved in 10% sodium bicarbonate (200 ml) and acetone (100 ml). 37.5 g (0.1 mol) of (Z)-5-fluoro-2-methyl-1-[(4-methylsulfinyl) phenylmethylene]-1H-indene-3-acetyl chloride was added into the reaction mixture. The mixture was stirred for 3 hours at RT. The solvents were evaporated off. The residue was suspended in ethyl acetate (500ml). 5% sodium bicarbonate (200 ml) was added into the reaction mixture with stirring. Ethyl acetate layer was collected and washed with water (3 x 500 ml). The ethyl acetate solution was dried over anhydrous sodium sulfate. Sodium sulfate was removed by filtration. 6 g of acetic acid was added into the reaction mixture with stirring. The organic solution was evaporated off. After drying, it yielded 42 g of the desired product (81.5%). Hygroscopic product; Solubility in water: 400 mg/ml; Elementary analysis: C₂₈H₃₄FNO₅S; MW: 515.64. Calculated% C: 65.22; H: 6.65; F: 3.68; N: 2.72; O: 15.51; 6.22; Found % C: 65.20; H: 6.67; N: 3.67; O: 15.53; S: 6.21. H-NMR (400 MHz, D₂O): δ: δ: 1.36 (t, 6H), 1.65 (s, 3H), 2.11 (s, 3H), 2.56 (s, 3H), 2.82 (s, 2H), 3.27 (m, 4H), 3.47(m, 2H), 4.48 (t, 2H), 6.74 (s, 1H), 6.84 (m,1H), 6.88 (b, 1H), 7.00 (m, 1H), 7.30 (m, 1H), 7.63 (m, 2H), 7.65 (m, 2H).

### Preparation of diethylaminoethyl

### 1-methyl-5-(4-methylbenzoyl)-1H-pyrrole-2-acetate.AcOH.

28.6 g (0.1 mol) of 1-methyl-5-(4-methylbenzoyl)-1H-pyrrole-2-acetyl chloride was dissolved in 100 ml of chloroform. The mixture was cooled to 0°C. 15 ml of triethylamine and 8.9 g (0.1 mol) of dimethylaminoethanol were added into the reaction mixture. The mixture was stirred for 3 hours at RT. The solvents were evaporated off. The residue was dissolved in methanol (300ml), 5% sodium bicarbonate (200 ml) was added into the reaction mixture. The mixture was stirred for 3 hr. The mixture was evaporated to dryness. Methanol (300 ml) was added into the residue with stirring. Solid was removed by filtration and washed with methanol. The solution was evaporated to dryness and the residue was dissolved in chloroform (200 ml). 6 g of acetic acid was added into the reaction mixture with stirring. Some solid was removed by filtration. Another 6 g of acetic acid was added into the reaction mixture with stirring. The organic solution was evaporated off. After drying, it yielded 37 g of the desired product (88.8%). Hygroscopic product; Solubility in water: 500 mg/ml; Elementary analysis: C₂₃H₃₂N₂O₅; MW: 416.51.Calculated% C: 66.32; H: 7.74; N: 6.73; O: 19.21; Found% C: 66.29; H: 7.76; N: 6.73; O: 19.22. ¹H-NMR (400 MHz, D₂O): δ: 1.51 (t, 6H), 2.21 (s, 3H), 2.35 (s, 3H), 3.25 (m, 4H), 3.48 (s, 3H), 3.50 (m, 2H), 3.55 (s, 2H), 4.50 (t, 2H), 5.86 (m, 1H), 6.67(m, 1H), 6.70 (b, 1H), 7.25 (m, 2H), 7.67 (m, 2H).

### Preparation of S-dimethylaminoethyl

### 1-(p-chlorobenzoyl)-5-methoxy-2-methylindole 3-acetate.AcOH

10.4 g (0.1 mol) of dimethyl amino ethyl mercaptan was dissolved in 10% sodium bicarbonate (200 ml) and acetone (100 ml). 37.6 g (0.1 mol) of 1-(p-chlorobenzoyl)-5-methoxy-2-methylindole 3-acetyl chloride was added into the reaction mixture. The mixture was stirred for 3 hours at RT. The solvents were evaporated off. The residue was suspended in ethyl acetate (500ml). 5% sodium bicarbonate (200 ml) was added into the reaction mixture with stirring. Ethyl acetate layer was collected and washed with water (3 x 500 ml). The ethyl acetate solution was dried over anhydrous sodium sulfate. Sodium sulfate was removed by filtration. 6 g of acetic acid was added into the reaction mixture with stirring. The organic solution was evaporated off. After drying, it yielded 46 g of the desired product (86.3%). Hygroscopic product; Solubility in water: 400 mg/ml; Elementary analysis: C₂₇H₃₃CIN₂O₅S; MW: 533.08. Calculated %C: 60.83; H: 6.24; Cl: 6.65; N: 5.26; O: 15.01; S: 6.02. Found % C: 60.80; H: 6.26; Cl: 6.66; N: 5.25; O: 15.02; S: 6.01. ¹H-NMR (400 MHz, D₂O): δ: 1.47 (t, 6H), 2.20 (s,3H), 2.28 (s, 3H), 3.28 (m, 4H), 3.31(t, 2H), 3.54 (s, 2H), 3.73 (s, 3H), 3.91 (t, 2H), 6.70 (b, 1H), 6.24 (m, 1H), 6.68 (m, 1H), 6.75 (m, 1H), 7.46 (m, 2H), 7.75 (m, 2H).

### Preparation of N-dimethylaminoethyl

### 5-(4-Chlorobenzoyl)-1,4-dimethyl-1H-pyrrole-2-acetamide.AcOH

8.8 g (0.1 mol) of dimethylaminoethylamine was dissolved in 10% sodium bicarbonate (200 ml) and acetone (100 ml). 31 g (0.1 mol) of 2-(3-benzoyphenyl) propionyl chloride was added into the reaction mixture. The mixture was stirred for 3 hours at RT. The solvents were evaporated off. The residue was suspended in ethyl acetate (500ml). 5% sodium bicarbonate (200 ml) was added into the reaction mixture with stirring. Ethyl acetate layer was collected and washed with water (3 x 500 ml). The ethyl acetate solution was dried over anhydrous sodium sulfate. Sodium sulfate was removed by filtration. 6 g of acetic acid was added into the reaction mixture with stirring. The organic solution was evaporated off. After drying, it yielded 33 g of the desired product (85.9 %). Hygroscopic product; Solubility in water: 400 mg/ml; Elementary analysis: C₂₃H₃₂CIN₃O₄; MW: 449.97. Calculated% C: 61.39; H: 7.17; Cl: 7.88; N: 9.34; O: 14.22; Found % C: 61.37; H: 7.18; Cl: 7.89; N: 9.32; O: 14.24. ¹H-NMR (400MHz, D₂O): δ: 1.42 (t, 6H), 2.05 (s, 3H), 2.21 (s, 3H), 3.26(m, 4H), 3.48 (s, 2H), 3.50(t, 2H), 3.64 (t, 2H), 3.50 (s, 3H), 5.66 (s, 1H), 7.0 (b, 1H), 7.46 (m, 2H), 7.54 (m, 2H),7.47 (b, 1H).

### Preparation of N-dimethylaminoethyl

### 1,8-diethyl-1,3,4,9-tetrahydropyrano-[3,4-b]indole-1-acetamide.AcOH

28.9 g (0.1 mol) of 1,8-diethyl-1,3,4,9-tetrahydropyrano-[3,4-b]indole-1-acetic acid was dissolved in 100 ml of acetonitrile. 32.1 g of O-(Benzotriazol-1-yl)-N,N,N',N'-tetramethyluronium tetrafluoroborate and 30 ml of triethylamine were added into the reaction mixture. 11.7 g of dimethylaminoethylamine was added into the reaction mixture. The mixture was stirred for 3 hours at RT. The solvents were evaporated off. 250 ml of ethyl acetate was added into the reaction mixture and the mixture was washed with water (3 x 100 ml). The organic solution was dried over anhydrous sodium sulfate. Sodium sulfate was removed by filtration. 6 g of acetic acid was added into the reaction mixture with stirring. Hexane (200 ml) was added. The solid product was collected by filtration. After drying, it yielded 40 g of the desired product (89.4%). Hygroscopic product; Solubility in water: 400 mg/ml; Elementary analysis: C₂₅H₄₁N₃O₄; MW: 447.61. Calculated% C: 67.08; H: 9.23; N: 9.39; O: 14.30; Found% C: 67.05; H: 9.25; N: 9.38; O: 14.32. ¹H-NMR (400 MHz, D₂O): δ: 0.95 (t, 3H), 1.20 (t, 3H), 1.24 (t, 3H), 1.40 (m, 2H), 1.50 (s, 6H), 1.87 (m, 2H), 2.21 (s, 3H), 2.25 (s, 2H), 2.56 (m, 2H), 3.08 (m, 1H), 3.11 (m, 1H), 3.28 (m, 4H), 3.50 (m, 2H), 3.64 (m, 2H), 7.0 (b, 1H), 6.43 (m, 1H), 6.70 (m, 1H), 6.72 (m, 1H), 7.6 (b,1H).

### Preparation of diethylaminoethyl 3-chloro-4-(2-propenyloxy) benzeneacetate.AcOH

60 g of Polymer-bound triethylamine (3 mol/g, 100-200 mesh) was suspended in 180 ml of chloroform. 22.7 g (0.1 mol) of 3-chloro-4-(2-propenyloxy) benzeneacetic acid was added into the mixture with stirring. 43 g (0.15mol) of diethylaminoethyl bromide.HBr was added into the mixture and the mixture was stirred for 5 hours at RT. The polymer was removed by filtration and washed with tetrahydrofuran (3 x 50 ml). 8.2 g (0.1 mol) of sodium acetate was added into the reaction mixture with stirring. The mixture was stirred for 2 h. The solid was removed by filtration and washed with chloroform (3 x 50 ml). The solution was concentrated in vacuo to 100 ml. Then 300 ml of hexane was added into the solution. The solid product was collected by filtration and washed with hexane (3 x 100 ml). After drying, it yielded 34 g of the desired product (88.3%). Hygroscopic product; Solubility in water: 400 mg/ml; Elementary analysis: C₁₉H₂₉ClN₂O₄; MW: 384.9. Calculated % C: 59.29; H: 7.59; Cl: 9.21, N: 7.28; O: 16.63; Found% C: 59.26; H: 7.61; Cl: 9.22; N: 7.26; O: 16.65. ¹H-NMR (400 MHz, D₂O: δ: 1.56 (t, 6H), 2.21 (s, 3H), 3.27 (m, 4H), 3.43 (s, 2H), 3.50 (m, 2H), 3.63 (m, 2H), 4.60 (s, 2H), 5.23 (m, 2H), 5.89 (m, 1H), 6.5 (b, 1H), 6.61 (m, 1H), 6.81 (m, 1H), 6.94 (m, 1H), 7.70 (b, 1H).

### Industrial Applicability

The pro-drugs presently disclosed are superior to tolmetin, zomepirac, etodolac, amfenac, bromofenac, alclofenac, fenclofenac, acemetacin, indomethacin, sulindac, fentiazac, lonazolac, bendazac, 6MNA, ibufenac, and related compounds. They may be used medicinally in a method for treating any tolmetin, zomepirac, etodolac, amfenac, bromofenac, alclofenac, fenclofenac, acemetacin, indomethacin, sulindac, fentiazac, lonazolac, bendazac, 6MNA, ibufenac and related compounds-treatable conditions in humans or animals. They may be used in a method for relieving signs and symptoms of rheumatoid arthritis and osteoarthritis, the reduction of fever, and in a method for treating dysmenorrhea. They may be also prescribed for diabetic neuropathy and acute migraine headache. Due to their very high membrane penetration rate, these pro-drugs. can be used in a method for treating asthma by inhalation to a host. They can be used in a method for treating acne due to their anti-inflammatory properties.

## Claims

1. A compound selected from the group consisting of:
Diethylaminoethyl 1-(p-chlorobenzoyl)-5-methoxy-2-methylindole 3-acetate.AcOH,
Diethylaminoethyl (Z)-5-fluoro-2-methyl-1-[(4-methylsulfinyl) phenylmethylene]-1H-indene-3-acetate.AcOH,
Diethylaminoethyl 1-methyl-5-(4-methylbenzoyl)-1H-pyrrole-2-acetate.AcOH,
Diethylaminoethyl 5-(4-Chlorobenzoyl)-1,4-dimethyl-1 H-pyrrole-2-acetate.AcOH,
Diethylaminoethyl 1,8-diethyl- 1,3,4,9-tetrahydropyrano-[3 ,4-b]indole-1-acetate.AcOH,
Diethylaminoethyl 2-amino-3-benzoylbenzeneacetate.AcOH,
Diethylaminoethyl 2-amino-3-(4-bromo-benzoyl)benzeneacetate.AcOH,
Diethylaminoethyl 3-chloro-4-(2-propenyloxy)benzeneacetate.AcOH,
Diethylaminoethyl 2-(2,4-dichlorophenoxy)benzeneacetate.AcOH,
Diethylaminoethyl 1-(4-chlorobenzoyl-5-methoxy-2-methyl-1H-indole-3-acetoxyacetate.AcOH,
Diethylaminoethyl 4-(4-chlorophenyl)-2-phenyl-5-thiazoleacetate.AcOH,
Diethylaminoethyl 3 -(4-chlorophenyl)-1-phenyl-1 H-pyrazole-4-acetate.AcOH,
Diethylaminoethyl [(1-benzyl- 1H-indazol-3-yl)oxy]acetate.AcOH,
Diethylaminoethyl 6-methoxyl-2-naphthalene-2-acetate.AcOH, and
Diethylaminoethyl p-isobutylphenylacetate.AcOH.

2. Process for the preparation of a compound according to claim 1, wherein acid halides or mixed anhydrides of tolmetin, zomepirac, etodolac, amfenac, bromofenac, alclofenac, fenclofenac, acemetacin, fentiazac, indomethacin, sulindac, lonazolac, bendazac, 6MNA or ibufenac are reacted with compounds of the general Structure (3) 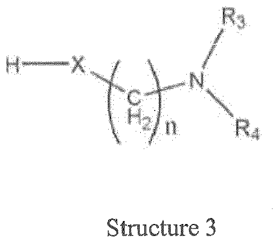 wherein, in structure 3, R₃ represents ethyl; R₄ represents ethyl; X represents O; and n = 2.

3. Process for the preparation of a compound according to claim 1, wherein tolmetin, zomepirac, etodolac, amfenac, bromofenac, alclofenac, fenclofenac, acemetacin, fentiazac, indomethacin, sulindac, lonazolac, bendazac, 6MNA or ibufenac are reacted with a compound of the general Structure (3) 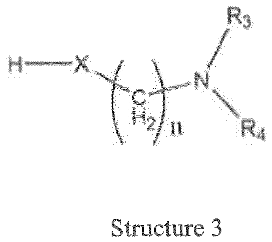 wherein, in structure 3, R₃ represents ethyl; R₄ represents ethyl; X represents O; and n = 2, by using coupling reagents.

4. The process of claim 3, wherein the coupling reagents are selected from the group consisting of N,N'-Dicyclohexylcarbodiimide, N, N'-Diisopropylcarbodiimide, O-(Benzotriazol-1-yl)-N,N,N',N'-tetramethyluronium tetrafluoroborate, O- (Benzotriazol-1-yl)-N,N,N',N'-tetramethyluronium hexafluorophosphate and Benzotriazol-1-yl-oxy-tris (dimethylamino)phosphonium hexafluorophosphate.

5. Process for the preparation of a compound according to claim 1, wherein metal salts, organic base salts or immobilized base salts of tolmetin, zomepirac, etodolac, amfenac, bromofenac, alclofenac, fenclofenac, acemetacin, fentiazac, indomethacin, sulindac, lonazolac, bendazac, 6MNA, ibufenac, and related compounds are reacted with compounds of the general Structure 4, 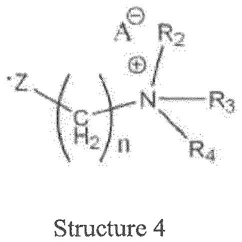 wherein, in structure 4, R₂ represents H, R₃ represents ethyl; R₄ represents ethyl; Z represents halogen or p-toluenesulphonyl, A⁻ represents any negative ion, and n = 2.

6. Composition comprising at least one compound according to claim 1.

7. Compound according to claim 1 or a pharmaceutical composition according to claim 6 for use in a method for treating a non-steroidal anti-inflammatory agent (NSAIA)-treatable condition selected from the group consisting of pain from a toothache, headache, and arthritis and other inflammatory pain, fever, cancer, dysmenorrhea, radiation-induced vomiting, diabetic neuropathy and acute migraine headache, hemophilic arthropathy, bone loss, and sunburn in humans or animals.

8. The compound or composition for use according to claim 7, wherein said uses comprises administering the compound or composition transdermally to any part of the body to deliver therapeutically effective plasma levels of the compound.

9. The compound or composition for use according to claim 7, wherein the NSAIA-treatable condition is selected from the group consisting of headache, toothache, and muscle pain, and arthritis and other inflammatory pain and comprising administering to the inflamed area a therapeutically effective amount of the compound or composition.

10. The compound or composition for use according to claim 7, wherein the NSAIA-treatable condition is selected from the group consisting of acne, sunburn or other skin disorders, wherein the compound or composition is administered transdermally and is in the form of a solution, spray, lotion, ointment, emulsion or gel.

11. The compound or composition for use according to claim 7, wherein the NSAIA-treatable condition is asthma, wherein the compound or composition is administered by spraying to through the mouth or nose or other parts of body.

12. The compound or composition for use according to claim 7, wherein the NSAIA-treatable condition is selected from the group consisting of eye inflammatory diseases, ocular pain after corneal surgery, glaucoma, and ear inflammatory and/or painful conditions (otitis).

13. Transdermal therapeutic application system comprising at least one compound according to claim 1 as an active ingredient for use in a method for treating an NSAIA-treatable conditions in humans or animals.

14. The transdermal therapeutic application system according to claim 13, wherein the systems is a bandage or patch comprising one active substance-containing matrix layer and an impermeable backing layer or wherein the system is an active substance reservoir, which has a permeable bottom facing the skin.

## Patentansprüche

1. Eine Verbindung ausgewählt aus der Gruppe bestehend aus:
Diethylaminoethyl-1-(p-chlorobenzoyl)-5-methoxy-2-methylindol-3-acetat.AcOH,
Diethylaminoethyl-(Z)-5-fluoro-2-methyl-1-[(4-methylsulfinyl)-phenylmethylen]-1H-inden-3-acetat.AcOH,
Diethylaminoethyl-1-methyl-5-(4-methylbenzoyl)-1H-pyrrol-2-acetat.AcOH,
Diethylaminoethyl-5-(4-Chlorobenzoyl)-1,4-dimethyl-1H-pyrrol-2-acetat.AcOH,
Diethylaminoethyl-1,8-diethyl- 1,3,4,9-tetrahydropyrano-[3,4-b]indol-1-acetat.AcOH,
Diethylaminoethyl-2-amino-3-benzoylbenzolacetat.AcOH,
Diethylaminoethyl-2-amino-3-(4-bromobenzoyl)benzolacetat.AcOH,
Diethylaminoethyl-3-chloro-4-(2-propenyloxy)benzolacetat.AcOH,
Diethylaminoethyl-2-(2,4-dichlorophenoxy)benzolacetat.AcOH,
Diethylaminoethyl-1-(4-chlorobenzoyl-5-methoxy-2-methyl-1H-indol-3-acetoxyacetat.AcOH,
Diethylaminoethyl-4-(4-chlorophenyl)-2-phenyl-5-thiazolacetat.AcOH,
Diethylaminoethyl-3-(4-chlorophenyl)-1-phenyl-1 H-pyrazol-4-acetat.AcOH,
Diethylaminoethyl-[(1-benzyl- 1H-indazol-3-yl)oxy]acetat.AcOH,
Diethylaminoethyl-6-methoxyl-2-naphthalin-2-acetat.AcOH, und
Diethylaminoethyl-p-isobutylphenylacetat.AcOH.

2. Ein Verfahren zur Herstellung einer Verbindung gemäß Anspruch 1, wobei azide Halogenide oder gemischte Anhydride von Tolmetin, Zomepirac, Etodolac, Amfenac, Bromofenac, Alclofenac, Fenclofenac, Acemetacin, Fentiazac, Indomethacin, Sulindac, Lonazolac, Bendazac, 6MNA oder Ibufenac mit Verbindungen der allgemeinen Struktur (3) umgesetzt werden, 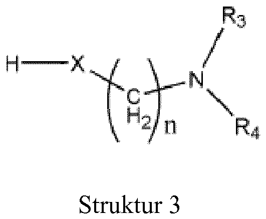 wobei, in Struktur 3, R₃ Ethyl darstellt; R₄ Ethyl darstellt; X O darstellt; und n = 2.

3. Verfahren zur Herstellung einer Verbindung gemäß Anspruch 1, wobei Tolmetin, Zomepirac, Etodolac, Amfenac, Bromofenac, Alclofenac, Fenclofenac, Acemetacin, Fentiazac, Indomethacin, Sulindac, Lonazolac, Bendazac, 6MNA oder Ibufenac mit einer Verbindung der allgemeinen Struktur (3) umgesetzt werden 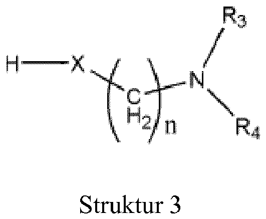 wobei, in Struktur 3, R₃ Ethyl darstellt; R₄ Ethyl darstellt; X O darstellt; und n = 2, durch Verwendung von Kopplungsreagenzien.

4. Das Verfahren gemäß Anspruch 3, wobei die Kopplungsreagenzien ausgewählt sind aus der Gruppe bestehend aus N,N'-Dicyclohexylcarbodiimid, N, N'-Diisopropylcarbodiimid, O-(Benzotriazol-1-yl)-N,N,N',N'-tetramethyluroniumtetrafluoroborat, O-(Benzotriazol-1-yl)-N,N,N',N'-tetramethyluroniumhexafluorophosphat und Benzotriazol-1-yl-oxy-tris (dimethylamino)phosphoniumhexafluorophosphat.

5. Verfahren zur Herstellung einer Verbindung gemäß Anspruch 1, wobei Metallsalze, organische basische Salze, oder immobilisierte basische Salze von Tolmetin, Zomepirac, Etodolac, Amfenac, Bromofenac, Alclofenac, Fenclofenac, Acemetacin, Fentiazac, Indomethacin, Sulindac, Lonazolac, Bendazac, 6MNA, Ibufenac und verwandten Verbindungen mit Verbindungen der allgemeinen Struktur 4 umgesetzt werden, 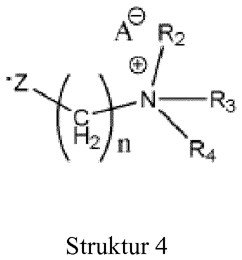 wobei, in Struktur 4, R₂ H darstellt; R₃ Ethyl darstellt; R₄ Ethyl darstellt; Z Halogen oder p-Toluolsulfonyl darstellt, A⁻ irgendein negatives Ion darstellt, und n = 2.

6. Zusammensetzung umfassend mindestens eine Verbindung gemäß Anspruch 1.

7. Verbindung gemäß Anspruch 1 oder eine pharmazeutische Zusammensetzung gemäß Anspruch 6 zur Verwendung in einem Verfahren zur Behandlung einer Erkrankung, die mit einem nicht-steroidalen, entzündungshemmenden Wirkstoff (NSAIA) behandelbar ist, ausgewählt aus der Gruppe bestehend aus Zahnschmerzen, Kopfschmerzen, Arthritis-bedingten Schmerzen und anderen entzündungsbedingten Schmerzen, Fieber, Krebs, Menstruationsbeschwerden, strahlungsbedingtem Erbrechen, diabetischer Neuropathie und akuter Migräne, hämophilischer Arthropathie, Knochenschwund und Sonnenbrand in Menschen oder Tieren.

8. Die Verbindung oder Zusammensetzung zur Verwendung gemäß Anspruch 7, wobei die Verwendungen die transdermale Verabreichung der Verbindung oder der Zusammensetzung an irgendeinen Teil des Körpers umfasst, um therapeutisch wirksame Plasmalevel der Verbindung zu erzielen.

9. Die Verbindung oder Zusammensetzung gemäß Anspruch 7, wobei die NSAIA-behandelbare Erkrankung ausgewählt ist aus der Gruppe bestehend aus Kopfschmerzen, Zahnschmerzen und Muskelschmerzen und Arthritis und anderen entzündungsbedingten Schmerzen und die Verabreichung einer therapeutisch wirksamen Menge der Verbindung oder Zusammensetzung an den entzündeten Bereich umfasst.

10. Die Verbindung oder Zusammensetzung zur Verwendung gemäß Anspruch 7, wobei die NSAIA-behandelbare Erkrankung ausgewählt ist aus der Gruppe bestehend aus Akne, Sonnenbrand oder anderen Hauterkrankungen, wobei die Verbindung oder Zusammensetzung transdermal verabreicht wird und in Form einer Lösung, eines Sprays, einer Lotion, einer Salbe, einer Emulsion oder eines Gels ist.

11. Die Verbindung oder Zusammensetzung zur Verwendung gemäß Anspruch 7, wobei die NSAIA-behandelbare Erkrankung Asthma ist, wobei die Verbindung oder Zusammensetzung durch Sprühen durch den Mund oder die Nase oder andere Körperteile verabreicht wird.

12. Die Verbindung oder Zusammensetzung zur Verwendung gemäß Anspruch 7, wobei die NSAIA-behandelbare Erkrankung ausgewählt ist aus der Gruppe bestehend aus entzündlichen Augenerkrankungen, Augenschmerzen nach Hornhautoperationen, Glaukom und entzündlichen und/oder schmerzhaften Ohrenerkrankungen (Otits).

13. Transdermales therapeutischen Verabreichungssystem umfassend mindestens eine Verbindung gemäß Anspruch 1 als einen aktiven Inhaltsstoff zur Verwendung in einem Verfahren zur Behandlung einer NSAIA-behandelbaren Erkrankung in Menschen oder Tieren.

14. Das transdermale therapeutische Verabreichungssystem gemäß Anspruch 13, wobei das System ein Verband oder Pflaster ist, umfassend eine Wirkstoff-enthaltende Matrixschicht und eine undurchlässige Stützschicht oder wobei das System ein Wirkstoff-Reservoir ist, das eine durchlässige, auf der Haut aufliegende Unterseite hat.

## Revendications

1. Composé choisi dans le groupe constitué de :
1-(p-chlorobenzoyl)-5-méthoxy-2-méthylindole-3-acétate de diéthylaminoéthyle.AcOH,
(Z)-5-fluoro-2-méthyl-1 -[(4-méthylsulfinyl)phénylméthylène]-1 H-indène-3-acétate de diéthylaminoéthyle.AcOH,
1-méthyl-5-(4-méthylbenzoyl)-1 H-pyrrole-2-acétate de diéthylaminoéthyle.AcOH, 5-(4-chlorobenzoyl)-1,4-diméthyl-1H-pyrrole-2-acétate de diéthylaminoéthyle.AcOH,
1,8-diéthyl-1,3,4,9-tétrahydropyrano-[3,4-b]indole-1-acétate de diéthylaminoéthyle.AcOH,
2-amino-3-benzoylbenzèneacétate de diéthylaminoéthyle.AcOH,
2-amino-3-(4-bromo-benzoyl)benzèneacétate de diéthylaminoéthyle.AcOH,
3-chloro-4-(2-propényloxy)benzèneacétate de diéthylaminoéthyle.AcOH,
2-(2,4-dichlorophénoxy)benzèneacétate de diéthylaminoéthyle.AcOH,
1-(4-chlorobenzoyl-5-méthoxy-2-méthyl-1 H-indole-3-acétoxyacétate de diéthylaminoéthyle.AcOH,
4-(4-chlorophényl)-2-phényl-5-thiazoleacétate de diéthylaminoéthyle.AcOH,
3-(4-chlorophényl)-1-phényl-1 H-pyrazole-4-acétate de diéthylaminoéthyle.AcOH,
[(1-benzyl- 1H-indazol-3-yl)oxy]acétate de diéthylaminoéthyle.AcOH,
6-méthoxyl-2-naphtalène-2-acétate de diéthylaminoéthyle.AcOH, et p-isobutylphénylacétate de diéthylaminoéthyle.AcOH.

2. Procédé de préparation d'un composé selon la revendication 1, dans lequel des halogénures d'acide ou d'anhydrides mixtes de tolmétine, zomépirac, étodolac, amfénac, bromofénac, alclofénac, fenclofénac, acémétacine, fentiazac, indométhacine, sulindac, lonazolac, bendazac, 6MNA ou ibufénac réagissent avec des composés de structure générale (3) 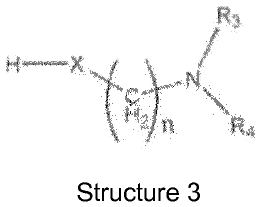 où, dans la structure 3, R₃ représente éthyle ; R₄ représente éthyle ; X représente O ; et n = 2.

3. Procédé de préparation d'un composé selon la revendication 1, dans lequel les tolmétine, zomépirac, étodolac, amfénac, bromofénac, alclofénac, fenclofénac, acémétacine, fentiazac, indométhacine, sulindac, lonazolac, bendazac, 6MNA ou ibufénac réagissent avec un composé de structure générale (3) 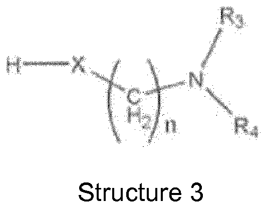 où, dans la structure 3, R₃ représente éthyle ; R₄ représente éthyle ; X représente O ; et n = 2, en utilisant des réactifs de couplage.

4. Procédé de la revendication 3, dans lequel les réactifs de couplage sont choisis dans le groupe constitué des N,N'-dicyclohexylcarbodiimide, N,N'-diisopropylcarbodiimide, tétrafluoroborate de O-(benzotriazol-1-yl)-N,N,N',N'-tétraméthyluronium, hexafluorophosphate de O-(benzotriazol-1-yl)-N,N,N',N'-tétraméthyluronium et hexafluorophosphate de benzotriazol-1-yl-oxy-tris(diméthylamino)phosphonium.

5. Procédé de préparation d'un composé selon la revendication 1, dans lequel des sels de métal, des sels de base organique ou des sels de base immobilisés de tolmétine, zomépirac, étodolac, amfénac, bromofénac, alclofénac, fenclofénac, acémétacine, fentiazac, indométhacine, sulindac, lonazolac, bendazac, 6MNA, ibufénac et des composés associés réagissent avec des composés de structure générale 4, 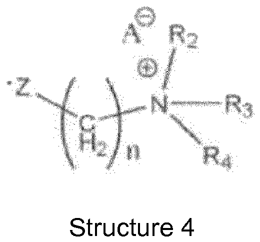 où, dans la structure 4, R₂ représente H, R₃ représente éthyle ; R₄ représente éthyle ; Z représente halogène ou p-toluènesulfonyle, A⁻ représente un ion négatif, et n = 2.

6. Composition comprenant au moins un composé selon la revendication 1.

7. Composé selon la revendication 1 ou composition pharmaceutique selon la revendication 6 pour utilisation dans un procédé pour traiter une affection pouvant être traitée par un agent anti-inflammatoire non stéroïdien (AINS) choisie dans le groupe constitué de la douleur causée par un mal de dent, un mal de tête et l'arthrite et une autre douleur inflammatoire, la fièvre, un cancer, une dysménorrhée, des vomissements induits par rayonnement, la neuropathie diabétique et des céphalées de migraine, l'arthropathie hémophilique, la perte osseuse et les coups de soleil chez des humains ou des animaux.

8. Composé ou composition pour utilisation selon la revendication 7, où lesdites utilisations comprennent l'administration du composé ou de la composition par voie transdermique à une partie quelconque du corps pour administrer des taux plasmatiques thérapeutiquement efficaces du composé.

9. Composé ou composition pour utilisation selon la revendication 7, où l'affection pouvant être traitée par un AINS est choisie dans le groupe constitué d'une céphalée, un mal de dent, et une douleur musculaire et l'arthrite et une autre douleur inflammatoire, et comprenant l'administration à la zone enflammée d'une quantité thérapeutiquement efficace du composé ou de la composition.

10. Composé ou composition pour utilisation selon la revendication 7, où l'affection pouvant être traitée par un AINS est choisie dans le groupe constitué de l'acné, un coup de soleil ou d'autres troubles cutanés, où le composé ou la composition est administré par voie transdermique et est sous la forme d'une solution, un aérosol, une lotion, une pommade, une émulsion ou un gel.

11. Composé ou composition pour utilisation selon la revendication 7, où l'affection pouvant être traitée par un AINS est l'asthme, où le composé ou la composition est administré par pulvérisation dans la bouche ou le nez ou sur d'autres parties du corps.

12. Composé ou composition pour utilisation selon la revendication 7, où l'affection pouvant être traitée par un AINS est choisie dans le groupe constitué de maladies inflammatoires oculaires, une douleur oculaire après chirurgie cornéenne, un glaucome, et des affections inflammatoires et/ou douloureuses auriculaires (otite).

13. Système d'application thérapeutique transdermique comprenant au moins un composé selon la revendication 1 en tant que substance active pour utilisation dans un procédé pour traiter des affections pouvant être traitées par un AINS chez des humains ou des animaux.

14. Système d'application thérapeutique transdermique selon la revendication 13, **caractérisé en ce que** le système est un bandage ou un patch comprenant une couche de matrice contenant une substance active et une couche de support imperméable ou que le système est un réservoir de substance active, qui a un fond perméable faisant face à la peau.
